(19) Europäisches Patentamt / European Patent Office / Office européen des brevets

(11) **EP 2 799 496 B1**

(12) **EUROPEAN PATENT SPECIFICATION**

(45) Date of publication and mention
of the grant of the patent:
**07.08.2019 Bulletin 2019/32**

(51) Int Cl.:
*A61L 27/44* (2006.01)      *A61K 6/087* (2006.01)
*C08J 3/20* (2006.01)      *C08K 9/06* (2006.01)

(21) Application number: **12856727.8**

(22) Date of filing: **11.12.2012**

(86) International application number:
**PCT/JP2012/082001**

(87) International publication number:
**WO 2013/089078 (20.06.2013 Gazette 2013/25)**

(54) **Dental material, bone substitute material and methods for their manufacturing**

Zahnmaterial, Knochenersatzmaterial und Verfahren zu ihrer Herstellung

Matériau dentaire, matériau de substituts osseux et procédés de leurs fabrications

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2011 JP 2011274731**
**15.12.2011 JP 2011274733**

(43) Date of publication of application:
**05.11.2014 Bulletin 2014/45**

(73) Proprietor: **Tokuyama Dental Corporation**
**Tokyo 110-0016 (JP)**

(72) Inventor: **YAMAGAWA, Jun-ichiro**
**Tokyo 110-0016 (JP)**

(74) Representative: **Gill Jennings & Every LLP**
**The Broadgate Tower**
**20 Primrose Street**
**London EC2A 2ES (GB)**

(56) References cited:
**EP-A1- 2 792 712      JP-A- 2003 171 120**
**JP-A- 2005 325 296      JP-A- 2008 137 854**

• DATABASE CA [Online] CHEMICAL ABSTRACTS SERVICE, COLUMBUS, OHIO, US; 19 June 2008 (2008-06-19), SHIMIZU, SHUJI ET AL: "Surface-treated uniform fine silica particles, manufacture thereof, and resin compositions containing them", XP002741559, retrieved from STN Database accession no. 2008:729281 -& JP 2008 137854 A (NIPPON CATALYTIC CHEM IND) 19 June 2008 (2008-06-19)
• DATABASE CA [Online] CHEMICAL ABSTRACTS SERVICE, COLUMBUS, OHIO, US; 17 June 2003 (2003-06-17), MIZOE, TOSHIYUKI: "Aluminum hydroxide for resin filler and its use in composition for artificial marble", XP002741560, retrieved from STN Database accession no. 2003:460430 -& JP 2003 171120 A (SUMITOMO CHEMICAL CO) 17 June 2003 (2003-06-17)
• DATABASE CA [Online] CHEMICAL ABSTRACTS SERVICE, COLUMBUS, OHIO, US; 2 December 2005 (2005-12-02), SHIBUYA, NOBUHIRO: "Thermoplastic resin compositions with no heat discoloration and good dimensional stability, and their moldings", XP002741561, retrieved from STN Database accession no. 2005:1265142 -& JP 2005 330378 A (MITSUBISHI PLASTICS IND) 2 December 2005 (2005-12-02)

• DATABASE CA [Online] CHEMICAL ABSTRACTS SERVICE, COLUMBUS, OHIO, US; 24 November 2005 (2005-11-24), SHIBUYA, NOBUHIRO: "Thermoplastic polymer compositions with low thermal expansion coefficient and improved tensile elongation, and their plastic films", XP002741562, retrieved from STN Database accession no. 2005:1240354 -& JP 2005 325296 A (MITSUBISHI PLASTICS IND) 24 November 2005 (2005-11-24)

**Description**

Technical Field

The present invention relates to a dental material comprising a resin composite, a manufacturing method of a dental material comprising a resin composite, a bone substitute material comprising a resin composite and a manufacturing method for a bone substitute material comprising a resin composite.

Background Art

A thermoplastic resin is excellent in mechanical properties, electrical properties, lightness in weight, molding processability, and the like, and hence is used in a wide range of fields such as electric/electronic/OA equipment, vehicles, building materials, agricultural materials, and miscellaneous goods. Further, incorporation of an inorganic filler is widely and generally performed as a method of improving rigidity, strength, dimensional characteristic, and the like of the thermoplastic resin. However, the following is a general tendency: physical properties of interest are improved by compositing the thermoplastic resin with the inorganic filler, but on the other hand, some other physical properties are sacrificed.

According to general classification, resin materials (plastics) are broadly classified into a thermoplastic resin and a thermosetting resin. The thermoplastic resin is a resin that is softened and melted when heated, thereby being able to be deformed into an arbitrary shape by a stress. Because of its easy processability and high productivity, the thermoplastic resin is applied in an extremely wide range of fields. On the other hand, the thermosetting resin has a feature in that the thermosetting resin becomes a network polymer after molding, thus being not softened by heating, resulting in high stability of a product but in poor productivity thereof. Therefore, in industry, the thermosetting resin is not applied as widely as the thermoplastic resin. In addition, the thermosetting resin is difficult to reprocess once a crosslinked structure is formed therein. Accordingly, a precursor thereof such as a polymerizable monomer is used as a product in many cases. For example, as a precursor material of a resin composite having a filler, such as inorganic particles, dispersed in a resin matrix, there have been proposed various composite compositions each having a filler, which is surface-treated with a silane coupling agent or the like, added in a polymerizable monomer (Patent Literatures 1 to 4 and the like). In each of those composite compositions, the filler is subjected to surface treatment for the purpose of, for example, improving an affinity between the polymerizable monomer and the filler, suppressing thixotropic property of the composite composition to secure workability, or improving dispersibility of the filler in the polymerizable monomer. In addition, each of those composite compositions is produced by subjecting the filler to the surface treatment and then mixing the treated filler with the polymerizable monomer, and is used by being polymerized and cured to provide a resin composite. Such surface treatment has been becoming more and more important from the viewpoint of surface control of a nanoparticle having a large specific surface area on a background of development of nanotechnology in recent years.

In addition, as for a resin composite using the thermoplastic resin, there is known a production method involving directly mixing a heated and melted resin with a filler. For example, there is a proposal of a resin composite containing 5 to 50 parts by mass of a filler surface-treated with an organosilicon compound with respect to 100 parts by mass in total of a thermoplastic polyimide resin and a polyarylketone resin (Patent Literature 5). It is described that in this resin composite, the surface treatment agent (organosilicon compound) undergoes some chemical change under a high-temperature melt-kneading condition to act on a surface condition of the filler so as to enhance its affinity for the resin, and exhibits a high effect on prevention of a marked change in hue of the resin composite even when the resin composite is exposed to heat treatment under high temperature for a long period of time.

In addition, there is also a proposal of a method of obtaining a melt-kneaded product, involving stirring a filler and a molten elastomer or resin while applying a shear force with a rotating screw (Patent Literature 6). The invention disclosed in Patent Literature 6 is one made on the basis of the assumption that in order to uniformly disperse a nanoscale filler, it is necessary and best to perform treatment by using an external factor to create a field for effective treatment means that surpasses an aggregating force of the filler while the following approach is insufficient: (1) an approach involving enhancing an affinity and interaction between the resin and the filler by a chemical technique such as modification of the filler; or (2) an approach involving adding an aggregation inhibitor to prevent chemical aggregation between particles of the filler.

[PTL 7] relates to the use of surface treated silica particles as fillers for various polymers.

[PTL 8] relates to the use of alumina particles which have been treated with organosilanes as fillers for vinyl ester resins.

[PTL 9] relates to a thermoplastic resin composition having organosilane surface treated fillers.

Citation List

Patent Literature

[PTL 1] JP 8-12305 A (for example, paragraphs 0037 and 0038)

[PTL 2] WO 2002/005752 A1 (for example, pages 24 and 25)

[PTL 3] JP 2005-170813 A (for example, paragraphs 0038 and 0039)

[PTL 4] JP 10-130116 A (for example, paragraph 0022)

[PTL 5] JP 2005-330378 A (for example, claim 1 and paragraph 0025)

[PTL 6] JP 2008-266577 A (for example, claim 1 and paragraph 0011)

[PTL 7] JP 2008-137854 A

[PTL 8] JP 2003-171120 A

[PTL 9] JP 2005-325296 A

Summary of Invention

Technical Problem

Resin composites obtained by curing the composite compositions disclosed in Patent Literatures 1 to 4, and the resin composites disclosed in Patent Literatures 5 and 6 each contain a filler dispersed in a resin matrix, and hence various mechanical characteristics can be easily improved. However, such resin composite is liable to undergo the elimination of the filler in terms of abrasion resistance, and is liable to be brittle because of containing the filler, with the result that its fracture energy is liable to be low.

However, when a resin composite is produced using a thermosetting resin, it is easy to construct a strong organic-inorganic hybrid structure by introducing an organic group to be used for a reaction with the thermosetting resin to surfaces of inorganic particles to be utilized as a filler by means of a silane coupling agent or the like, thereby forming chemical bonding between the resin matrix and the surfaces of the inorganic particles. For example, a method involving introducing an epoxy group or an amino group to the surfaces of inorganic particles to be used in combination with an epoxy resin is generally well known.

On the other hand, a thermoplastic resin molecule has no reaction-capable organic functional group in many cases, and even if having a reaction-capable organic functional group, has the reaction-capable organic functional group only at an end portion of its polymer chain. That is, in the thermoplastic resin molecule, the presence ratio of the reaction-capable organic functional group is extremely low. Accordingly, when a resin composite is produced using the thermoplastic resin, it is difficult to construct a strong organic-inorganic hybrid structure through the formation of a sufficient amount of chemical bonding between the resin matrix and the inorganic particles. Against such background, in the production of a composite having a filler added to a thermoplastic resin, surface treatment of the filler is designed with a main purpose of improving the affinity of the filler for the resin as described above.

The present invention has been made in view of the above-mentioned circumstances, and an object of the present invention is to provide: a resin composite that while exploiting the high productivity and high chemical resistance and color fastness of a thermoplastic resin, is excellent in abrasion resistance and has high rigidity, dimensional stability, mechanical strength, and fracture energy, as compared to a related-art resin composite; a dental material and a bone substitute material using the resin composite; and a manufacturing method for the resin composite.

Solution to Problem

The above-mentioned object is achieved by the present invention described below. That is, the present invention is as described below.

A dental or bone substitute material comprising a resin composite of the present invention is produced using:

(A) 100 parts by mass of a thermoplastic resin having a melting point or fluidization temperature of from 300°C to 500°C; and (B) 40 to 400 parts by mass of inorganic particles surface-treated with a surface treatment agent represented by the following general formula (I), and has an average embedding rate, which is represented by the following equation (1), of 55% or more:

•General formula (I)         $A\text{-}R^1\text{-}SiR^2mBn$

in the general formula (I): A represents an organic group having a polymerizable carbon-carbon double bond, or an aromatic group; $R^1$ represents an organic group having a straight chain moiety constituted of 3 to 12 atoms; $R^2$ represents a hydrocarbon group having 1 to 6 carbon atoms; B represents an alkoxy group containing a hydrocarbon having 1 to 6 carbon atoms, a halogen group, or an isocyanate group; and m and n each represent an integer, the sum of m and n is 3, and m represents an integer of from 0 to 2;

·Equation (1)      Average embedding rate (%)=$100\times\{EP/(EP+V)\}$

in the equation (1): EP represents the number {particles/(10 $\mu$m$\times$10 $\mu$m)} of the inorganic particles per square region measuring 10 $\mu$m on each side exposed in a polished surface subjected to platinum vapor deposition treatment in a case where a polished surface formed by subjecting a test piece formed of the resin composite to microtome polishing using a diamond knife is subjected to the platinum vapor deposition treatment and then the polished surface subjected to the platinum vapor deposition treatment is observed with a scanning electron microscope; V represents the number {voids/(10 $\mu$m$\times$10 $\mu$m)} of voids per square region measuring 10 $\mu$m on each side formed through elimination of the inorganic particles in the square region measuring 10 $\mu$m on each side where the EP number is counted; and the average embedding rate refers to an average value of embedding rates respectively determined at four measurement points.

In a dental or bone substitute material according to one embodiment of the present invention, (A) the thermoplastic resin having a melting point or fluidization temperature of from 300°C to 500°C preferably includes an aromatic polyether ketone resin.

In a dental or bone substitute material according to another embodiment of the present invention, the aromatic polyether ketone resin preferably includes polyetheretherketone.

In a dental or bone substitute material according to another embodiment of the present invention, the surface treatment agent (B) preferably includes a surface treatment agent represented by the following general formula (II):

General formula (II)         $CH_2\text{=}C(R^3)\text{-}COO\text{-}(CH_2)_p\text{-}SiR^2mBn$

in the general formula (II), $R^3$ represents a hydrogen atom or a methyl group, p represents an integer of from 3 to 12, and $R^2$, B, m, and n have the same meanings as those shown in the general formula (I).

In a dental or bone substitute material according to another embodiment of the present invention, (B) the inorganic particles surface-treated with the surface treatment agent each preferably have an average particle diameter of 0.05 $\mu$m or more and 15 $\mu$m or less.

A bone substitute material of the present invention includes the resin composite of the dental or bone substitute material of the present invention.

A manufacturing method for manufacturing a dental or bone substitute material comprising a resin composite of the present invention includes at least a melt-kneading step of kneading raw materials including: (A) 100 parts by mass of a thermoplastic resin having a melting point or fluidization temperature of from 300°C to 500°C; and (B) 40 to 400 parts by mass of inorganic particles surface-treated with a surface treatment agent represented by the following general formula (I), while melting the raw materials by heating in the range of from the melting point or the fluidization temperature or more to 500°C or less:

General formula (I)         $A\text{-}R^1\text{-}SiR^2mBn$

in the general formula (I): A represents an organic group having a polymerizable carbon-carbon double bond, or an aromatic group; $R^1$ represents an organic group having a straight chain moiety constituted of 3 to 12 atoms; $R^2$ represents a hydrocarbon group having 1 to 6 carbon atoms; B represents an alkoxy group containing a hydrocarbon having 1 to 6 carbon atoms, a halogen group, or an isocyanate group; and m and n each represent an integer, the sum of m and n is 3, and m represents an integer of from 0 to 2.

In a method according to one embodiment of the present invention, (B) the surface treatment agent preferably includes a surface treatment agent represented by the following general formula (II):

General formula (II) $\qquad$ $CH_2=C(R^3)-COO-(CH_2)_p-SiR^2mBn$

in the general formula (II), $R^3$ represents a hydrogen atom or a methyl group, p represents an integer of from 3 to 12, and $R^2$, B, m, and n have the same meanings as those shown in the general formula (I).

In a method according to another embodiment of the present invention, (B) the inorganic particles surface-treated with the surface treatment agent each preferably have an average particle diameter of 0.05 $\mu$m or more and 15 $\mu$m or less.

Advantageous Effects of Invention

According to the present invention, it is possible to provide: a dental or bone substitute material comprising the resin composite that while exploiting the high productivity and high chemical resistance and color fastness of a thermoplastic resin, is excellent in abrasion resistance and has high rigidity, dimensional stability, mechanical strength, and fracture energy, as compared to a related-art resin composite; the dental material using the resin composite; and the manufacturing method for a dental or bone substitute material comprising the resin composite.

Description of Embodiments

A dental or bone substitute material comprising a resin composite according to this embodiment is produced using: (A) 100 parts by mass of a thermoplastic resin having a melting point or fluidization temperature of from 300°C to 500°C; and (B) 40 to 400 parts by mass of inorganic particles surface-treated with a surface treatment agent represented by the following general formula (I), and has an average embedding rate, which is represented by the following equation (1), of 55% or more.

General formula (I) $\qquad$ $A-R^1-SiR^2mBn$

Equation (1) $\qquad$ Average embedding rate (%)=$100\times\{EP/(EP+V)\}$

In the general formula (I): A represents an organic group having a polymerizable carbon-carbon double bond, or an aromatic group; $R^1$ represents an organic group having a straight chain moiety constituted of 3 to 12 atoms; $R^2$ represents a hydrocarbon group having 1 to 6 carbon atoms; B represents an alkoxy group containing a hydrocarbon having 1 to 6 carbon atoms, a halogen group, or an isocyanate group; and m and n each represent an integer, the sum of m and n is 3, and m represents an integer of from 0 to 2.

In addition, in the equation (1): EP represents the number {particles/(10 $\mu$m$\times$10 $\mu$m)} of the inorganic particles per square region measuring 10 $\mu$m on each side exposed in a polished surface subjected to platinum vapor deposition treatment in a case where a polished surface formed by subjecting a test piece formed of the resin composite to microtome polishing using a diamond knife is subjected to the platinum vapor deposition treatment and then the polished surface subjected to the platinum vapor deposition treatment is observed with a scanning electron microscope; V represents the number {voids/(10 $\mu$m$\times$10 $\mu$m)} of voids per square region measuring 10 $\mu$m on each side formed through elimination of the inorganic particles in the square region measuring 10 $\mu$m on each side where the EP number is counted; and the average embedding rate refers to an average value of embedding rates respectively determined at four measurement points.

It should be noted that the diamond knife to be used was one having a blade angle of 45° and a blade width of 2 mm or more. An example of such diamond knife is a diamond knife ultra 45° manufactured by DiATOME. In addition, the machining conditions are as follows: clearance angle: 6°, machining speed: 0.5 mm/sec, machined test piece thickness (feed length): 100 nm, and machined test piece area: 0.5 mm to 0.8 mm in width by 0.8 mm to 1.2 mm in length. In addition, the machining is performed by a wet process using, as water, distilled water. The machined surface of the main body after being machined 5 times counted from the beginning of uniform machining of at least the surface of the test piece under such conditions is used for observation. As the diamond knife, a new one, a repolished one, or a similarly sharp one with a non-damaged blade is used.

The sample that has been machined with the diamond knife is fixed onto a sample stage for a scanning electron microscope using a conductive adhesive such as a carbon tape or carbon paste, and a platinum film having a thickness of 10 nm is formed on the sample through vapor deposition under a vacuum with a sputter coater. As the scanning electron microscope, an apparatus using an electron gun of a field emission type (FE-SEM) and having a resolution of 2 nm or more is used in order to obtain a clear image. The measurement conditions are set to an accelerating voltage of 10 kV, a beam spot size of 3.0 nm, a sample tilt angle of 0°, and a working distance of 5 mm, and a reflected electron detector is used. Measurement is performed after adjusting the focus and astigmatism so that an image may become clearest, and adjusting the magnification so that the total of the number of particles and number of voids per SEM photograph (per observation region) may become from 100 to 300. A brighter reflected electron image is observed in the inorganic particles than in the resin, and hence the inorganic particles can be easily counted through appropriate adjustment of the brightness and contrast. In counting the number of inorganic particles and the number of voids, one should be careful not to count air bubbles contained from the beginning in the sample. The voids are easily distinguishable because the voids are directed in the direction of the machining with the diamond knife. In addition, two or more voids may merge to form a large void, and in such case, the large void is counted as the two or more voids in light of the sizes of the inorganic particles and the shapes of the voids.

It should be noted that the average embedding rate is more preferably 60% or more, still more preferably 65% or more.

In this context, the dental or bone substitute material comprising the resin composite according to this embodiment is produced through at least a melt-kneading step of kneading raw materials including: (A) 100 parts by mass of a thermoplastic resin having a melting point or fluidization temperature of from 300°C to 500°C; and (B) 40 to 400 parts by mass of inorganic particles surface-treated with a surface treatment agent represented by the general formula (I), while melting the raw materials by heating in the range of from the melting point or the fluidization temperature or more to 500°C or less: In this case, when the thermoplastic resin is a crystalline resin, a crystalline resin having a melting point of from 300°C to 500°C is appropriate, and when the thermoplastic resin is a non-crystalline resin, a non-crystalline resin having a fluidization temperature of from 300°C to 500°C is appropriate. With the thermoplastic resin having such melting point or fluidization temperature, the thermoplastic resin and the inorganic particles are melted and mixed at a heating temperature ("melting temperature") selected from the range of from the melting point or the fluidization temperature to 500°C. In the range of from the melting point or the fluidization temperature or more to 500°C or less, the thermoplastic resin has a temperature region in which its melt viscosity measured with a capillary viscometer, that is, its shear viscosity at a shear rate of 1,000 (1/s) exhibits a value in the range of preferably from 10 Pa·s to 10,000 Pa·s, more preferably from 250 Pa·s to 1,000 Pa·s. Such melt viscosity is excellent in ease of kneading of the thermoplastic resin and the inorganic particles, and in ease of molding of the resin composite to be obtained, and the melting temperature is preferably selected from such range. In general, such suitable melting temperature is selected from temperatures higher than the melting point or the fluidization temperature by from 10 to 50°C.

Accordingly, the dental or bone substitute material comprising the resin composite according to this embodiment, while exploiting the high productivity and high chemical resistance and color fastness of the thermoplastic resin, is excellent in abrasion resistance and has high rigidity, dimensional stability, mechanical strength, and fracture energy, as compared to a related-art resin composite using a thermoplastic resin. In this connection, when the thermoplastic resin having a melting point or fluidization temperature of from 200°C to 500°C is merely blended with the inorganic particles at the above-mentioned melting temperature, the resin composite itself becomes brittle and its fracture energy lowers, although the various effects listed above are easily obtained. In the resin composite according to this embodiment, however, the fracture energy can also be improved. Although the reason why such effect is obtained is not known in detail, the inventor of the present invention has the following assumption. That is, when the inorganic particles are dispersed in the resin matrix, the inorganic particles are eliminated from the surface of the resin composite relatively easily upon, for example, polishing, machining, or sliding of the surface. This is because the inorganic particles and the resin cannot form strong chemical bonding such as covalent bonding, and the only bonding force acting between the inorganic particles and the resin matrix is an intermolecular force whose anchoring effect on the inorganic particles is weak. In addition, as described above, in the case of a thermosetting resin, strong chemical bonding (covalent bonding) can be formed between the resin matrix and the inorganic particles through the utilization of a reactive group of a polymerizable monomer. In the case of the thermoplastic resin, however, it is difficult to form the strong covalent bonding as described above between the thermoplastic resin and the inorganic particles.

Accordingly, the only bonding force acting between the inorganic particles and the resin matrix formed of the thermoplastic resin is the intermolecular force. In addition, the same holds true for the resin composite exemplified in Patent Literature 6, which has inorganic particles dispersed in a resin matrix with only a high shear force. That is, it is considered that in such related-art resin composite using a thermoplastic resin, the bonding force at an interface between the resin matrix and the inorganic particles is extremely weak.

Therefore, when a force is applied to the inorganic particles upon, for example, polishing of the surface of such resin composite, the inorganic particles are easily eliminated from the resin matrix. In addition to this, when a stress is applied to the resin composite, stress concentration occurs at the interface between the resin matrix and the inorganic particles, where the bonding force is weakest. As a result, breakage easily occurs at the interface.

On the other hand, the dental or bone substitute material comprising the resin composite according to this embodiment uses the inorganic particles surface-treated with the surface treatment agent represented by the general formula (I) in its manufacture. It should be noted that in the following description, the inorganic particles that have been surface-treated with the surface treatment agent represented by the general formula (I) are referred as "surface-treated inorganic particles", and the original inorganic particles themselves are referred to simply as "inorganic particles".

Herein, A shown in the general formula (I) represents an organic group having a polymerizable carbon-carbon double bond, or an aromatic group. It should be noted that herein, when A shown in the general formula (I) represents the "organic group", the "organic group" may be an aliphatic organic group or may be an aromatic organic group. In addition, when A shown in the general formula (I) represents the "aromatic group", the "aromatic group" means only an aromatic group that does not contain a polymerizable carbon-carbon double bond such as a vinyl group bonded to a benzene ring.

When A shown in the general formula (I) represents an organic group having a polymerizable carbon-carbon double bond, the heating at the time of the melt-kneading causes carbon-carbon double bonds to react with each other to form a crosslink in a surface layer formed on the surfaces of the inorganic particles with the surface treatment agent. In addition, the number of atoms constituting the straight chain moiety of the organic group $R^1$, which determines the molecular chain length of the surface treatment agent, is 3 or more, and hence the molecular chain length of the surface treatment agent is sufficiently long to form entanglement with a molecule (polymer chain) of the thermoplastic resin. Therefore, the surface layer is caused to have a three-dimensional network structure in which the molecules of the surface treatment agent are further bonded to each other based on a structure in which individual molecules of the surface treatment agent are merely bonded to the surfaces of the inorganic particles. Accordingly, the individual polymer chains constituting the resin matrix are easily entangled with the network structure of the surface layer. In addition to this, a shear force to be applied at the time of the melt-kneading promotes the entanglement of the polymer chains with the network structure of the surface layer. Therefore, in the resin composite according to this embodiment, at the interface between the resin matrix and the surface-treated inorganic particles, a bonding force originating from the entanglement between the molecular chains, which is extremely strong as compared to an intermolecular force, acts in addition to a bonding force based on the intermolecular force.

In addition, the number of atoms constituting the straight chain moiety of the organic group $R^1$, which determines the molecular chain length of the surface treatment agent, is 3 or more, and hence the molecular chain length of the surface treatment agent is sufficiently long to penetrate between the polymer chains to form entanglement therewith. Further, the bulkiness of the organic group A is larger than the bulkiness of the organic group $R^1$, and hence it is estimated that the penetration of the organic group A between the polymer chains due to a shear force to be applied at the time of the melt-kneading facilitates the exhibition of a three-dimensional anchoring effect. On the other hand, it is expected that when the chain length of the organic group $R^1$ is excessively long, the network structure of the surface layer becomes sparse, with the result that the polymer chains may be hardly entangled with the network structure or the entanglement may be liable to be undone. However, the straight chain moiety of the organic group $R^1$ is constituted of 12 or less atoms, and hence it is considered that such problem can be suppressed.

In addition, when A shown in the general formula (I) represents an aromatic group, the aromatic group A is a group having large bulkiness and allowing the formation of a stacking structure through a $\pi$-$\pi$ interaction between the molecules of the surface treatment agent positioned on the surface. In addition, as described above, the number of atoms constituting the straight chain moiety of the organic group $R^1$ is 3 or more, and hence the molecular chain length of the surface treatment agent is sufficiently long to penetrate between the polymer chains to form entanglement therewith. Therefore, the individual polymer chains constituting the resin matrix are easily entangled with the molecules of the surface treatment agent constituting the surface layer. In addition to this, a shear force to be applied at the time of the melt-kneading promotes the entanglement of the molecules of the surface treatment agent and the polymer chains. Further, the bulkiness of the aromatic group A is larger than the bulkiness of the organic group $R^1$, and hence it is estimated that the penetration of the aromatic group A between the polymer chains due to the shear force to be applied at the time of the melt-kneading facilitates the exhibition of a three-dimensional anchoring effect. On the other hand, when the chain length of the organic group $R^1$ is excessively long, it is difficult to hold a long and thin polymer chain in such a manner that the polymer chain is sandwiched between the bulky aromatic group A and the surface of the inorganic particle, and hence it is expected that the above-mentioned three-dimensional anchoring effect remarkably lowers. However, it is considered that when the number of atoms constituting the straight chain moiety of the organic group $R^1$ is set to 12 or less, the lowering of the three-dimensional anchoring effect as described above can be suppressed.

In addition to the foregoing, it is estimated that the aromatic group A formed of an aromatic group can further strengthen the interaction with the individual polymer chains constituting the resin matrix through a $\pi$-$\pi$ interaction. That is, when the thermoplastic polymer constituting the resin matrix contains in the molecule a $\pi$ electron originating from an aromatic ring or the like, a bonding force based on an interaction between the $\pi$ electron on the polymer side and a $\pi$ electron of the aromatic group constituting the aromatic group A acts in addition to a usual bonding force based on an intermolecular force.

It should be noted that the action and effect described above are considered to be similarly exhibited also in the case where A shown in the general formula (I) represents an organic group having a polymerizable carbon-carbon double bond and the organic group is aromatic.

Therefore, in the resin composite according to this embodiment, even if a force is applied to the surface-treated inorganic particles upon, for example, polishing of the surface of the resin composite, easy elimination of the surface-treated inorganic particles from the resin matrix can be suppressed. In addition to this, when a stress is applied to the resin composite, breakage at an interface between the resin matrix and the surface-treated inorganic particles is suppressed even if the stress is concentrated at the interface.

Next, details of the components constituting the resin composite of the dental or bone substitute material according to this embodiment are described below.

(A) Thermoplastic resin of the dental or bone substitute material

As the thermoplastic resin of the dental or bone substitute material, a known thermoplastic resin may be utilized as long as the resin has a melting point or fluidization temperature of from 300°C to 500°C. Herein, the reason why the melting point or the fluidization temperature is set to 300°C or more is as follows: when melt-kneading is performed using the thermoplastic resin and the surface-treated inorganic particles, in the case where A shown in the general formula (I) represents an organic group, a temperature of at least 200°C or more is needed to perform a polymerization reaction between polymerizable carbon-carbon double bonds contained in the organic groups A. In addition, when the melting point or the fluidization temperature is set to 200°C or more, the heat resistance of the resin composite can be enhanced. In addition, it is preferred that the melting point or the fluidization temperature be high also from the viewpoints of: promoting the molecular motion of the surface treatment agent fixed to the surface of each of the inorganic particles to enhance reactivity with the adjacent surface treatment agent; and enabling sufficient mixing of the inorganic particles with the thermoplastic resin that has been melted after the formation of a network structure by the surface treatment agent on their surfaces. In addition, the upper limit value of the melting point or the fluidization temperature has only to be 500°C or less from the viewpoint of practical use such as ease of selecting and obtaining the material, is more preferably 430°C or less, still more preferably 400°C or less. In addition, the thermoplastic resin is preferably a linear thermoplastic resin, and its main chain preferably has a structure having an arylene group because of its high heat resistance and mechanical strength. In addition, the arylene group preferably has no substituent because the arylene group having no substituent is considered to allow effective formation of an entangled structure with the network structure of the surface layer covering the surfaces of the inorganic particles.

As the thermoplastic resin having the above-mentioned melting point or fluidization temperature, specifically, there may be used a thermoplastic resin containing: divalent organic groups each selected from an arylene group and an imide group; and a linking group selected from a carbonyl group, an ether group, a sulfide group, a sulfonyl group, an ester group, an amide group, and an isopropylidene group, the linking group linking the divalent organic groups together. In addition, as the thermoplastic resin, any of a fluorine-based thermoplastic resin and a non-fluorine-based thermoplastic resin may be used. Specific examples of the non-fluorine-based thermoplastic resin having a melting point or fluidization temperature of from 300 to 500°C include modified polyphenylene ether (PPE: suitable melting temperature: from 240 to 320°C), polyethylene terephthalate (PET: suitable melting temperature: from 220 to 350°C), polycarbonate (PC: suitable melting temperature from 250 to 320°C), polyphenylene sulfide (PPS: suitable melting temperature: from 310 to 340°C), polysulfone (PSF or PSU: suitable melting temperature: from 330 to 400°C), polyether sulfone (PES or PESU: melting temperature: from 310 to 400°C), polyphenylsulfone (PPSU: suitable melting temperature: from 360 to 390°C), polyarylate (PAR: suitable melting temperature: from 310 to 390°C), polyamide imide (PAI: suitable melting temperature: from 320 to 370°C), polyether imide (PEI: suitable melting temperature: from 340 to 430°C), a liquid crystal polymer (LCP: suitable melting temperature: from 320 to 400°C), and aromatic polyether ketone (suitable melting temperature: from 340 to 400°C). In addition, the molecular structure of the thermoplastic resin is preferably a linear structure having small steric hindrance, and the thermoplastic resin preferably has a low molecular weight and preferably has high fluidity. Each of these features is considered to contribute to the promotion of the formation of an entangled structure with the surface treatment agent covering the surfaces of the inorganic particles. Further, when A shown in the general formula (I) represents an aromatic group, the molecule of the thermoplastic resin preferably has a linear structure having small steric hindrance, and the thermoplastic resin preferably has a low molecular weight and preferably has high fluidity. Each of these features is considered to facilitate the exhibition of a three-dimensional anchoring effect through penetration between the molecules of the thermoplastic resin by the aromatic group A constituting the molecule of the surface treatment agent covering the surfaces of the inorganic particles at the time of the melt-kneading. The same holds true for the case where A shown in the general formula (I) represents an aromatic organic group having a polymerizable carbon-carbon double bond.

Of those resins, an aromatic polyether ketone resin, which has a structure in which benzene rings are linearly linked via linking groups (ether groups and ketone groups), is preferably used because the aromatic polyether ketone resin has particularly high mechanical strength (against compressive stress, tensile stress, fatigue, abrasion, and the like), chemical resistance, water resistance, and discoloration resistance. In this case, examples of the aromatic polyether ketone resin include: polyetherketone (PEK) having a basic linear structure in which an ether group and a ketone group are alternately arranged as linking groups for linking together benzene rings constituting a linear main chain, the benzene rings being adjacent to each other; polyetheretherketone (PEEK) in which linking groups are arranged in the following order: an ether group, an ether group, and a ketone group; polyetherketone ketone (PEKK) in which linking groups are arranged in the following order: an ether group, a ketone group, and a ketone group; polyether etherketoneketone (PEEKK) in which linking groups are arranged in the following order: an ether group, an ether group, a ketone group, and a ketone group; and polyetherketoneetherketoneketone (PEKEKK). It should be noted that of those polyether ketone resins, polyetheretherketone is preferably used. Each of those resins is linear and has no substituent on a benzene ring as well, and has a polymer chain having no bulky structure, and hence is considered to easily form an entangled structure with the surface treatment agent covering the surfaces of the inorganic particles. Further, when A shown in the general formula (I) represents an aromatic group, the exhibition of a three-dimensional anchoring effect is considered to be facilitated through penetration between the molecules of the thermoplastic resin by the aromatic group A constituting the molecule of the surface treatment agent covering the surfaces of the inorganic particles at the time of the melt-kneading. The same holds true for the case where A shown in the general formula (I) represents an aromatic organic group having a polymerizable carbon-carbon double bond. In addition, as the resin, two or more kinds of resins may be appropriately mixed and used. Further, as required, a thermoplastic resin other than those listed above may be appropriately used.

The molecular weight of the thermoplastic resin may be appropriately selected, but is preferably selected in consideration of points described below. In general, as the molecular weight of the thermoplastic resin increases, mechanical characteristics tend to improve, but the fluidity of the plasticized thermoplastic resin tends to lower, which may make it difficult to blend a large amount of the inorganic particles. In addition, as the fluidity of the plasticized thermoplastic resin is lower, an increased load is imposed on manufacturing apparatus in the melt-kneading step to be carried out in the manufacture of the dental or bone substitute material comprising the resin composite according to this embodiment, and in other various steps, such as an extrusion step and an injection molding step, to be carried out as required, and hence stable manufacture may be difficult. However, as the fluidity of the thermoplastic resin is lower, the efficiency of the kneading of the thermoplastic resin and the inorganic particles becomes higher and the average embedding rate can be made higher. Therefore, the molecular weight of the thermoplastic resin is preferably selected in consideration of a balance among those factors.

(B) Surface-treated inorganic particles

The inorganic particles constituting the surface-treated inorganic particles may be any known inorganic particles without any particular limitation. In this case, for example, the following materials may be specifically used as materials for the inorganic particles: amorphous silica, borosilicate glass, soda glass, aluminosilicate glass, fluoroaluminosilicate glass, and glass containing a heavy metal (such as barium, strontium, or zirconium); crystallized glass obtained by depositing a crystal in the glass, a glass ceramics such as crystallized glass obtained by depositing a crystal of diopside or leucite; a composite inorganic oxide such as silica-zirconia, silica-titania, or silica-alumina; an oxide obtained by adding an oxide of a Group I metal to the composite oxide; or an inorganic oxide of a metal such as silica, alumina, titania, or zirconia. The glass transition temperature, transformation temperature, and melting point of each of the inorganic particles are preferably higher than the molding temperature of the thermoplastic resin by 100°C or more.

Of the materials for the inorganic particles listed above, silica, or a metal composite oxide containing silica as a main component is particularly preferred because a functional group can be easily introduced through surface treatment utilizing a silane coupling reaction, and strong covalent bonding can be formed between the surface treatment agent and the inorganic particles. In this context, the main component means that a silica component is contained at 50 mass% or more, and it is preferred that the metal composite oxide contain silica at 80 mass% or more. In addition, in order to strengthen the interaction between the thermoplastic resin and the inorganic particles at their interface, an interfacial area, that is, the specific surface area of the inorganic particles is preferably $2.0 \text{ m}^2/\text{g}$ or more and less than $20 \text{ m}^2/\text{g}$, more preferably from $5.0 \text{ m}^2/\text{g}$ to $10.0 \text{ m}^2/\text{g}$. In addition, the shape of each of the particles is also not particularly limited, and shapes such as a truly spherical form, a substantially spherical form, a beaded form, a dimple form, a konpeito form, a cloud form, a grape form, and a fibrous form may be used. Most simply, irregularly shaped inorganic particles in a crushed form are suitably used.

The ratio at which active groups (e.g., metal hydroxide groups such as silanol groups) with which the surface treatment agent can react are present in the surfaces of the inorganic particles is preferably as high as possible. This is probably because a shorter distance between functional groups to be introduced through the surface treatment allows the construction of a stronger crosslinked structure. In order to manufacture such inorganic particles, the inorganic particles are preferably manufactured by performing firing at a relatively low temperature of from 750 to 950°C so that the number of silanol groups remaining on the surfaces may be as large as possible. For example, the sol-gel method exemplified in JP 58-110414 A, JP 58-156524 A, or the like may be suitably utilized as such manufacturing method for the inorganic particles. In addition, from the viewpoint of high reactivity of the surface treatment agent, it is more preferred to use inorganic particles each having a surface covered with silica so that a large number of silanol groups may be present as reaction-capable active groups. In this case, it is easy to use silica as a raw material, but silanol groups may be introduced by subjecting the surfaces of other inorganic particles to silica coating treatment. In this case, the thickness of a silica coating layer is preferably 3 nm or more as measured by transmission microscope observation. As a method of forming such silica coating, for example, the method disclosed in JP 2008-7381 A may be used.

It should be noted that in the case of utilizing the resin composite of the dental or bone substitute material according to this embodiment, silica, zirconia, and titanium dioxide are each suitable as a material for the inorganic particles because of, for example, ease of imparting transparency close to that of tooth substance and less harmfulness to a living body. The selection of zirconia as the inorganic particles is preferred because in this case, the modulus of elasticity and hardness of the resin composite according to this embodiment can be made higher than in the case of selecting any other inorganic particles, and the characteristics of a molded body can be made closer to those of a metal material. In addition, the shape of each of the inorganic particles is not particularly limited, and may be appropriately selected from a spherical shape, a substantially spherical shape, an irregular shape, a needle shape, an aggregate shape, a cluster shape, and the like. In general, it is preferred to use spherical or substantially spherical inorganic particles or powder obtained by aggregating the spherical or substantially spherical inorganic particles through heat treatment or the like.

In addition, the surfaces of the inorganic particles are subjected to surface treatment with the surface treatment agent represented by the general formula (I). A, which constitutes the surface treatment agent, is formed of an organic group having a polymerizable carbon-carbon double bond, or an aromatic group. A preferably represents an organic group having a polymerizable carbon-carbon double bond in order to more easily obtain a resin composite excellent in abrasion resistance and having high rigidity, dimensional stability, mechanical strength, and fracture energy, as compared to a related-art resin composite.

In this case, examples of the organic group A having a polymerizable carbon-carbon double bond include: i) an unsaturated aliphatic group including, for example, a vinyl group, a (meth)acrylic group, or a (meth)acrylamide group; and ii) an aromatic group such as a phenyl group, a phenoxy group, a phenylamino group, a benzophenone group, a hydroxybenzophenone group, a biphenyl group, or a naphthyl group, the aromatic group including a vinyl group, a (meth)acrylic group, or a (meth)acrylamide group as its substituent. It should be noted that when the organic group A of the surface treatment agent represented by the general formula (I) is formed of the above-mentioned aromatic group, an interaction with each individual polymer chain constituting the resin matrix is estimated to become stronger through a $\pi$-$\pi$ interaction. That is, when the thermoplastic polymer constituting the resin matrix contains in the molecule a $\pi$ electron originating from an aromatic ring or the like, a bonding force based on an interaction between the $\pi$ electron on the polymer side and a $\pi$ electron of the aromatic group constituting the organic group A acts in addition to a usual bonding force based on an intermolecular force.

In addition, examples of the aromatic group A include: a monocyclic hydrocarbon group having one aromatic ring such as a phenyl group, a phenoxy group, a phenylamino group, a benzophenone group, or a hydroxybenzophenone group; a non-condensed polycyclic hydrocarbon group having two aromatic rings such as a biphenyl group; and a condensed polycyclic hydrocarbon group having two aromatic rings such as a naphthyl group. It should be noted that the non-condensed polycyclic hydrocarbon group means a group in which a carbon atom constituting one aromatic ring and a carbon atom constituting the other aromatic ring form direct bonding. In addition, those aromatic groups A do not include a substituent having a polymerizable carbon-carbon double bond such as a vinyl group, a (meth)acrylic group, or a (meth)acrylamide group.

$R^1$ represents an organic group having a straight chain moiety constituted of 3 to 12 atoms, and particularly preferably represents a linear one, not a branched one having a side chain moiety, in order to facilitate the formation of an entangled structure with the molecules of the thermoplastic resin. In addition, for the same reason, the number of atoms constituting the straight chain moiety is preferably from 3 to 6, most preferably from 4 to 6. When the chain length of $R^1$ is excessively short, an entangled structure with the molecules of the thermoplastic resin is hardly formed, and in the case where A shown in the general formula (I) represents an aromatic organic group A having a polymerizable carbon-carbon double bond, or an aromatic group A, a three-dimensional anchoring effect is not obtained. In addition, when the chain length of $R^1$ is excessively long, a sufficient reinforcing effect is hardly obtained. It should be noted that the atoms constituting the straight chain moiety of the organic group $R^1$ are generally constituted only of carbon atoms, but may include a heteroatom such as an oxygen atom for forming an ether linkage. In this context, examples of the organic group $R^1$ in the case where the straight chain moiety is constituted of 3 atoms may include $-CH_2-CH_2-CH_2-$ and $-CH_2-CH_2-O-$. A typical example of $R^1$ is a linear alkylene group (including the case where at least one of the carbon atoms constituting the main chain is substituted by a heteroatom such as an oxygen atom).

m may represent an integer of 0, 1, or 2. In this regard, however, when m is small (that is, n is large), a surface three-dimensional structure is complicated owing to a condensation reaction between silanol groups. Consequently, a satisfactory entangled structure is hardly formed, and in the case where A shown in the general formula (I) represents an aromatic organic group A having a polymerizable carbon-carbon double bond, or an aromatic group A, a three-dimensional anchoring effect is hardly exhibited. Therefore, it is preferred that m=1 (n=2).

Examples of such surface treatment agent may include 11-methacryloyloxyundecyltrimethoxysilane, 11-methacryloyloxyundecylmethyldimethoxysilane, 10-methacryloyloxydecyltrimethoxysilane, 10-methacryloyloxydecylmethyldimethoxysilane, 10-methacryloyloxydecyltrichlorosilane, 8-methacryloyloxyoctyltrimethoxysilane, 8-methacryloyloxyoctylmethyldimethoxysilane, 8-methacryloyloxyoctyldimethylmethoxysilane, 8-methacryloyloxyoctyltrichlorosilane, 6-methacryloyloxyhexylmethyldimethoxysilane, 4-methacryloyloxybutyltrimethoxysilane, γ-methacryloy-loxypropyltrimethoxysilane, γ-methacryloyloxypropylmethyldimethoxysilane, γ-methacryloyloxypropyldimethylmethox-ysilane, γ-methacryloyloxypropyltrichlorosilane, γ-methacryloyloxypropyltriisocyanatosilane, γ-methacryloyloxypropyldimethylisocyanatosilane, γ-methacryloyloxypropyltriethoxysilane, 3-(4-methacryloyloxyphenyl)propyltrimethoxysilane, 3-(4-methacryloyloxyphenyl)propyltrichlorosilane, 3-(4-methacryloyloxyphenyl)propyltriisocyanatosilane, styrylpropyltrimethoxysilane, 3-(N-styrylmethyl-2-aminoethylamino)-propyltrimethoxysilane, (methacryloyloxymethyl)phenylbutyltrimethoxysilane, O-(methacryloxyethyl)-N-(triethoxysilylpropyl)carbamate, N-(3-methacryloxy-2-hydroxypropyl)-3-aminopropyltriethoxysilane, and acrylate compounds corresponding to these methacrylate compounds, phenoxypropyl-trichlorosilane, phenoxypropylmethyldichlorosilane, phenoxypropyldimethylchlorosilane, benzoylpropyltrimethoxysi-lane, phenylaminopropyltrimethoxysilane, 3-phenylpropylmethyldichlorosilane, 4-phenylbutyltrichlorosilane, 4-phenylbutylmethyldichlorosilane, 11-phenoxyundecyltrichlorosilane, 2-hydroxy-4-(3-triethoxysilylpropoxy)diphenyl ketone, 6-phenylhexyldimethylchlorosilane, N-1-phenylethyl-N'-triethoxysilylpropylurea, 3-(4-methacryloyloxyphenyl)propyltrimethoxysilane, 3-(4-methacryloyloxyphenyl)propyltrichlorosilane, 3-(4-methacryloyloxyphenyl)propyltriisocyanatosilane, styrylpropyltrimethoxysilane, 3-(N-styrylmethyl-2-aminoethylamino)-propyltrimethoxysilane, and (methacryloyloxymethyl)phenylbutyltrimethoxysilane.

In order that the surface treatment agent may form a regular structure on the surfaces of the inorganic particles, it is preferred that the surfaces of the inorganic particles be covered with a single layer of the surface treatment agent. Although it is difficult to cover the surfaces of the inorganic particles with a single layer of the surface treatment agent in an exact sense, the optimum amount of the surface treatment agent can be roughly estimated on the basis of a relationship between the minimum coverage area with the surface treatment agent and the specific surface area of the inorganic particles. That is, the optimum amount of the surface treatment agent (theoretical amount) can be calculated by dividing a value, which is obtained by multiplying the weight of the inorganic particles by the specific surface area of the inorganic particles, by the minimum coverage area with the surface treatment agent. In addition, the amount of the surface treatment agent preferably falls within the range of from 0.7 to 1.2 relative to the theoretical amount.

It should be noted that the surface treatment agent particularly preferably contains in one molecule at least one (meth)acrylic group, and at least one reactive functional group for forming bonding to the surfaces of the inorganic particles. Such surface treatment agent is not particularly limited, but a surface treatment agent having a reactive functional group at one end of a linear molecule, and having a (meth)acrylic group at the other end is preferably used. Specifically, a surface treatment agent represented by the following general formula (II) is particularly preferred.

•General formula (II)　　　　　$CH_2=C(R^3)-COO-(CH_2)_p-SiR^2{}_mB_n$

In the general formula (II), $R^3$ represents a hydrogen atom or a methyl group, p represents an integer of any one of from 3 to 12. $R^2$, B, m, and n have the same meanings as those shown in the general formula (I).

Examples of the silane coupling agent represented by the general formula (II) may include 11-methacryloylox-yundecyltrimethoxysilane, 11-methacryloyloxyundecylmethyldimethoxysilane, 10-methacryloyloxydecyltrimethoxysi-lane, 10-methacryloyloxydecylmethyldimethoxysilane, 10-methacryloyloxydecyltrichlorosilane, 8-methacryloyloxyoctyltrimethoxysilane, 8-methacryloyloxyoctylmethyldimethoxysilane, 8-methacryloyloxyoctyldimethylmethoxysilane, 8-methacryloyloxyoctyltrichlorosilane, 6-methacryloyloxyhexylmethyldimethoxysilane, 4-methacryloyloxybutyltrimethoxysilane, γ-methacryloyloxypropyltrimethoxysilane, γ-methacryloyloxypropylmethyldimethoxysilane, γ-methacryloyloxypropyldimethylmethoxysilane, γ-methacryloyloxypropyltrichlorosilane, γ-methacryloyloxypropyltriisocyanatosilane, γ-methacryloyloxypropyldimethylisocyanatosilane, γ-methacryloyloxypropyltriethoxysilane, and acrylate compounds corresponding to these methacrylate compounds.

One kind of the surface treatment agents may be used alone, or two or more kinds thereof may be used as a mixture. In addition, when the surfaces of the inorganic particles are subjected to surface treatment with the surface treatment agent, the surface treatment agent is preferably used at a ratio of from 1 part by mass to 10 parts by mass with respect to 100 parts by mass of the inorganic particles. As a method for the surface treatment, a known method may be used without any particular limitation. Examples thereof include: a method involving adding the surface treatment agent by spraying while vigorously stirring the inorganic particles with an stirring blade or the like; a method involving dispersing the inorganic particles in an appropriate solvent, dissolving the surface treatment agent therein, and then removing the solvent; a method involving converting an alkoxy group of the surface treatment agent into a silanol group through hydrolysis with an acid catalyst in an aqueous solution, to thereby attach the surface treatment agent to the surfaces of the inorganic particles in the aqueous solution, and then removing water by a method such as spray drying; and a method involving dispersing the inorganic particles in an appropriate dispersion medium, dissolving the surface treatment agent therein, performing reflux, and then recovering the inorganic particles by filtration and classification. It should be noted that in any of the methods, in general, when heating is performed in the range of preferably from 50 to 200°C, more preferably from 100°C to 150°C, a reaction between the surfaces of the inorganic particles and the surface treatment agent can be promoted, and surface treatment with high durability can be performed.

In addition, the average particle diameter of each of the surface-treated inorganic particles (or the inorganic particles) is not particularly limited, but falls within the range of preferably from 0.05 $\mu$m or more to 15 $\mu$m or less, more preferably from 0.2 $\mu$m or more to 5 $\mu$m or less, most preferably from 0.3 $\mu$m or more to 0.6 $\mu$m or less. When the average particle diameter is 0.05 $\mu$m or more, the surface-treated inorganic particles can be easily dispersed and incorporated into the resin matrix through the prevention of the aggregation of the surface-treated inorganic particles. In addition, when the average particle diameter is set to 15 $\mu$m or less, the material homogeneity of the resin composite as a whole can be kept. It should be noted that when two or more kinds of surface-treated inorganic particles are used, the average particle diameters of the respective surface-treated inorganic particles may be identical to or different from each other, but each average particle diameter preferably falls within the range of from 0.05 $\mu$m or more to 15 $\mu$m or less. It should be noted that the term "average particle diameter" as used herein refers to a D50 value in terms of volume fraction in measurement with a particle size distribution analyzer.

In addition, the blending amount of the surface-treated inorganic particles with respect to 100 parts by mass of the resin falls within the range of from 40 parts by mass to 400 parts by mass, preferably from 60 parts by mass to 300 parts by mass, more preferably from 80 parts by mass to 150 parts by mass. When the blending amount is set to 40 parts by mass or more, as in a related-art resin composite, improved mechanical strength and excellent dimensional stability can be obtained, and moreover, as the blending amount of the surface-treated inorganic particles is larger, the efficiency of kneading can be improved to promote the interface reaction more, resulting in a higher average embedding rate. In addition, when the blending amount is set to 400 parts by mass or less, it is easy to prevent aggregation between the surface-treated inorganic particles or to suppress dispersion failure.

(C) Other components

It should be noted that other components may be appropriately added to the resin composite depending on the intended use of the resin composite. For example, an antistatic, a UV absorber, a pigment, a colorant, and the like may be added.

(Method for manufacturing a dental or bone substitute material comprising a resin composite)

As described above, the dental or bone substitute material comprising the resin composite according to this embodiment is manufactured through at least the melt-kneading step of kneading raw materials including the thermoplastic resin and the surface-treated inorganic particles while melting the raw materials by heating at from 300°C to 500°C. An apparatus to be used in the melt-kneading step is not particularly limited as long as it is a known melt-kneading apparatus. For example, a mixer with a heating apparatus, a single-screw melt-kneading apparatus, or a twin-screw melt-kneading apparatus may be used. As the apparatus to be used in the melt-kneading step, a twin-screw extrusion molding machine is suitably used. Although the specifications, screw shape, and operational conditions of the twin-screw extrusion molding machine may be arbitrarily selected depending on purposes, as a screw rotation number increases and a pressure applied to a kneaded product increases, the average embedding rate of the surface-treated inorganic particles can be made higher. This is probably because an interface reaction between the thermoplastic resin and the surface-treated inorganic particles proceeds, and entanglement of molecular chains between the molecules of the thermoplastic resin and the surface layer of the surface-treated inorganic particles proceeds more effectively.

In addition, after the melt-kneading step, various post-processes may be carried out as required. For example, the melt-kneaded product in a high-temperature state immediately after the melt-kneading step may be directly subjected to injection molding, extrusion molding, or the like to be molded into a given shape. In addition, the melt-kneaded product in a high-temperature state immediately after the melt-kneading step may be first molded into a member for secondary processing having a pellet shape, a powder shape, a block shape, or the like, and then the member for secondary processing may be further subjected to any of various types of processing such as injection molding, extrusion molding, laser forming, cutting processing, machining processing, and polishing processing.

In the manufacture of the dental or bone substitute material comprising the resin composite according to this embodiment, after the melt-kneading step, in general, a molded body having a given shape is obtained by any of various molding methods such as injection molding, extrusion molding, and compression molding. At this time, the productivity of the molded body can be enhanced by performing rapid cooling in the mold. In such case, the rapid cooling may cause a residual stress inside the molded body. In addition, in the case of using a crystalline resin as the thermoplastic resin, the crystal structure of the crystalline resin may not be formed in an ideal manner. In order to solve those problems, in the manufacturing method for the resin composite according to this embodiment, the obtained molded body may be subjected to heat treatment as required. When the heat treatment is performed, a residual stress inside the molded body can be released. In addition, when a crystalline resin is used as the thermoplastic resin, the recrystallization of the resin vitrified by the rapid cooling can be promoted by the heat treatment, and as a result, the mechanical strength of the molded body can be enhanced.

A method for the heat treatment (heat treatment step) is not particularly limited, but the temperature is preferably selected from a temperature region of the glass transition temperature or more where the melt viscosity is not exceeded, and is preferably selected from the range of from 150 to 300°C. The heat treatment time is preferably selected from the range of from 30 minutes to 6 hours. A cooling step after the heat treatment preferably involves leaving the molded body in a heating apparatus such as an oven in which the heat treatment has been performed under a state in which its heat source is turned off to return the temperature to room temperature over a period of time of 1 hour or more. In addition, for the same reason, when the molded body having a given shape is obtained by a molding method such as injection molding, extrusion molding, or compression molding, by carrying out the above-mentioned cooling step after the resin composite according to this embodiment has been molded into the given shape, it is also possible to obtain a finished molded article having high strength without performing the heat treatment step.

(Applications of resin composite)

The resin composite is utilized as a dental material. The resin composite according to this embodiment contains the inorganic particles in a large amount and has excellent mechanical strength, and hence can exhibit excellent mechanical durability when used as a dental material that is routinely exposed to a large pressure upon chewing in the mouth.

In addition to the foregoing, even when the surface of the resin composite according to this embodiment is exposed to sliding or friction by contact with a toothbrush or a tooth opposed to a tooth that uses the resin composite, the inorganic particles are hardly eliminated and the surface is hardly abraded. Accordingly, at a treatment site using the resin composite according to this embodiment, luster and smoothness at the time of the treatment can be maintained over a long period of time. In addition, therefore, an aesthetic appearance hardly deteriorates over a long period of time, rough feeling on the tongue can be suppressed, and surface irregularities due to the elimination of the inorganic particles are reduced. As a result, attachment of plaque and discoloration resulting therefrom can be suppressed.

The resin composite according to this embodiment is also utilized as a bone substitute material, which is required to have mechanical strength in most cases.

Examples

Hereinafter, the present invention is more specifically described by way of Examples.

First, thermoplastic resins used in Examples A1 to A32 and Comparative Examples A1 to A11 and their abbreviations, inorganic particles used therein and their abbreviations, surface treatment agents used therein and their abbreviations, and sample production methods and evaluation methods are shown below.

(Thermoplastic resin)

P1: polybutylene terephthalate (DURANEX 2000, Polyplastics Co., Ltd., melting point: 228°C, melting viscosity at 250°C: 150 Pa·s)

P2: polyetheretherketone resin (PEEK 1000G, Daicel-Evonik Ltd., melting point: 334°C, melting viscosity at 370°C: 200 Pa·S)

P3: polypropylene (BC8, Japan Polypropylene Corporation, melting point: 170°C, melting viscosity at 190°C: 150 Pa·S)

P4: polyetheretherketone resin (PEEK 381G, Victrex, melting point: 334°C, melting viscosity at 370°C: 400 Pa·S)

P5: polyetheretherketone resin (PEEK 90G, Victrex, melting point: 334°C, melting viscosity at 370°C: 100 Pa·S)

(Inorganic particles)

F1: average particle diameter: 0.40 micron, spherical sol-gel silica (Tokuyama Corporation), specific surface area:

7.0 m$^2$/g

F2: average particle diameter: 2.5 microns, crushed silica-zirconia (Tokuyama Corporation), specific surface area: 2.9 m$^2$/g

F3: average particle diameter: 2.8 microns, spherical silica (Tokuyama Corporation), specific surface area: 12 m$^2$/g

F4: average particle diameter: 0.15 micron, spherical silica-zirconia (Tokuyama Corporation), specific surface area: 22 m$^2$/g

F5: average particle diameter: 3.2 microns, talc (Nippon Talc Co., Ltd.), specific surface area: 12 m$^2$/g

F6: average particle diameter: 8.6 microns, zirconia (DAIICHI KIGENSO KAGAKU KOGYO CO., LTD.), aggregate having a primary particle diameter of 1.0 micron, specific surface area: 3.3 m$^2$/g

F7: average particle diameter: 4.0 microns, silica (Tokuyama Corporation), specific surface area: 1.3 m$^2$/g

(Surface treatment agent)

S5: 3-styrylpropyltrimethoxysilane (SPTMS)

S6: 3-(N-styrylmethyl-2-aminoethylamino)-propyltrimethoxysilane (SMAPTMS)

S7: γ-methacryloyloxypropyltrimethoxysilane (MPTMS)

S8: 4-methacryloyloxybutyltrimethoxysilane (MBTMS)

S9: 8-methacryloyloxyoctyltrimethoxysilane (MOTMS)

S10: 10-methacryloyloxydecylmethyldimethoxysilane (MDDMS)

S11: hexamethyldisilazane (HMDS)

S12: octadecyltrimethoxysilane (ODTMS)

S13: phenyltrimethoxysilane (PTMS)

S14: vinyltrimethoxysilane (VTMS)

S15: 1-phenyl-1-trichlorosilylbutane (PTCSB)

(Measurement method for bending strength and modulus of elasticity)

A compression-molded body having a thickness of 2 mm was cut at intervals of 2 mm using a rotary diamond cutter while water was poured to prepare five rod-shaped test pieces each having a length of 25 mm, a width of 2 mm, and a thickness of 2 mm for each sample. Burrs on the samples were removed using #800 waterproof abrasive paper, the width and thickness of the central portion of each test piece were measured with a micrometer, and a three-point bending test was performed with a universal testing machine AG-50kl (SHIMADZU CORPORATION) at room temperature in the air under the conditions of a support distance of 20 mm and a crosshead speed of 1 min/mm to prepare a stress (bending strength)-strain curve. At this time, bending strength was determined on the basis of the following equation (2).

$$\text{·Equation (2)} \qquad \sigma_B = 3PS/2WB^2$$

In the equation (2), $\sigma_B$ represents bending strength (Pa), P represents a load (N) at the time of the breakage of the test piece, S represents a support distance (m), W represents the width (m) of the test piece, and B represents the thickness (m) of the test piece.

In addition, a bending modulus of elasticity was determined on the basis of the following equation (3). A load of from 5 N to 10 N was used for the calculation.

$$\cdot \text{Equation (3)} \qquad E_B = (S^3/4WB^3) \times (F/Y)$$

In the equation, $E_B$ represents a bending modulus of elasticity (Pa), and F/Y represents the slope (N/m) of a load-deflection curve. In addition, S, W, and B have the same meanings as those shown in the equation (2).

(Measurement method for Vickers hardness)

The Vickers hardness of the surface of a compression-molded body was measured using a microhardnessmeter (manufactured by MATSUZAWASEIKI) under the conditions of a load of 100 gf and a loading time of 30 seconds. Measurement was performed at four points, and an average value thereof was determined.

(Calculation method for average embedding rate)

An end portion of a resin composite machined into a test piece measuring $2.0 \times 2.0 \times 10$ mm using a diamond cutter was adjusted with a laser blade so as to have a width of from 0.5 mm to 0.8 mm and a length of from 0.8 mm to 1.2 mm. After that, the end portion was machined using an ultramicrotome (manufactured by RMC) and a diamond knife ultra 45° (manufactured by DiATOME) under a distilled water-filled state under the conditions of a clearance angle of 6°, a machining speed of 0.5 mm/sec, and a machined test piece thickness of 100 nm. Then, the polished surface of the test piece after being machined at least 5 times counted from the beginning of uniform machining of the surface of the test piece was used for observation. On the polished surface of the thus machined test piece, a platinum film having a thickness of 10 nm was formed by vapor deposition treatment. Subsequently, the polished surface subjected to the platinum vapor deposition treatment was observed with a field emission scanning electron microscope (FE-SEM, resolution at 10 kV: 1.5 nm). The observation conditions were set to an accelerating voltage of 10 kV, a beam spot size of 3.0 nm, a sample tilt angle of 0°, and a working distance of 5 mm, and a reflected electron detector was used. Four images were photographed with the focus, astigmatism, contrast, and brightness adjusted so as to allow inorganic particles and voids to be clearly recognized. Then, an average embedding rate was determined by the following equation (1).

$$\cdot \text{Equation (1)} \qquad \text{Average embedding rate (\%)} = 100 \times \{EP/(EP+V)\}$$

In the equation (1), EP represents the number {particles/(10 $\mu$m$\times$10 $\mu$m)} of inorganic particles per square region measuring 10 $\mu$m on each side exposed in the polished surface, and V represents the number {voids/(10 $\mu$m$\times$10 $\mu$m)} of voids per square region measuring 10 $\mu$m on each side formed through the elimination of the inorganic particles in the square region measuring 10 $\mu$m on each side where the EP number is counted.

<Comparable Example A1>

A slurry was obtained by weighing and mixing 100 g of the inorganic particles F1 and 200 ml of toluene and then dispersing the mixture to a primary particle level with a homogenizer. The slurry was loaded into a three-necked flask having set thereon a reflux condenser tube, 2.4 g of the surface treatment agent S5 were added thereto, and the mixture was heated to reflux for 2 hours while being stirred. The solid content was separated with a centrifugal separator, washed twice with toluene, and then dried in a vacuum dryer at 90°C for 10 hours. Thus, surface-treated inorganic particles were obtained.

Next, 100 parts by mass of (A) the thermoplastic resin P1, and 80 parts by mass of (B) the surface-treated inorganic particles were weighed, and loaded into a kneader LABO PLASTOMILL (manufactured by Toyo Seiki Seisakusho, Ltd.). Kneading was performed at a test temperature (melting temperature) of 250°C and a rotation number of 100 rpm for 5 minutes, and then the sample was recovered. The sample was subjected to compression molding into a plate shape (50 mm in length$\times$50 in width$\times$2 mm in thickness) using a hot compression molding machine, and slowly cooled to provide a resin composite. Table 1 shows the raw material composition and evaluation results of the resin composite.

<Comparable Examples A2 to 6>

Surface-treated inorganic particles to be used for the production of resin composites were produced in the same manner as in Comparable Example A1 except that the conditions were changed as shown in Table 1. Subsequently, the resin composites were obtained by the same method as that of Comparable Example A1 except that the blending amount of the inorganic particles was set to a condition shown in Table 1. Table 1 shows the raw material composition and evaluation results of each of the resin composites.

<Examples A7 to 21>

Surface-treated inorganic particles to be used for the production of resin composites were produced in the same manner as in Comparable Example A1 except that the conditions were changed as shown in Table 1. Subsequently, the resin composites were obtained by the same method as that of Comparable Example A1 except that: the thermoplastic resin P2 was used in place of the thermoplastic resin P1; the blending amount of the inorganic particles was set to a condition shown in Table 1; and the test temperature (melting temperature) was set to 370°C. Table 1 shows the raw material composition and evaluation results of each of the resin composites.

<Example A22>

Surface-treated inorganic particles to be used for the production of a resin composite were produced in the same manner as in Comparable Example A1 except that the conditions were changed as shown in Table 1.
Next, 100 parts by mass of (A) the thermoplastic resin P2, and 150 parts by mass of (B) the surface-treated inorganic particles were weighed, and loaded into a small twin-screw extrusion molding machine (manufactured by Parker Corporation, Inc.). Kneading was performed under the conditions of a test temperature (melting temperature) of 370°C and a rotation number of 500 rpm, and then the sample was recovered. The sample was subjected to compression molding into a plate shape (50 mm in length×50 in width×2 mm in thickness) using a hot compression molding machine, and slowly cooled to provide a resin composite. Table 1 shows the raw material composition and evaluation results of the resin composite.

<Examples A23 to 32>

Surface-treated inorganic particles to be used for the production of resin composites were produced in the same manner as in Comparable Example A1 except that the conditions were changed as shown in Table 1. Subsequently, the resin composites were obtained by the same method as that of Example A1 except that: the thermoplastic resin used and the blending amount of the inorganic particles were set to conditions shown in Table 1; and the test temperature (melting temperature) was set to 370°C. Table 1 shows the raw material composition and evaluation results of each of the resin composites.

<Comparative Example A1>

A resin composite was obtained in the same manner as in Comparable Example A1 except that the blending amount of the inorganic particles was changed to 30 parts by mass. Table 2 shows the raw material composition and evaluation results of the resin composite.

<Comparative Examples A2 and 3>

Surface-treated inorganic particles to be used for the production of resin composites were produced in the same manner as in Comparable Example A1 except that the conditions were changed as shown in Table 1.
Next, 100 parts by mass of (A) the thermoplastic resin P1, and 80 parts by mass of (B) the surface-treated inorganic particles were weighed, and loaded into a kneader LABO PLASTOMILL (manufactured by Toyo Seiki Seisaku-sho, Ltd.). Kneading was performed at a test temperature (melting temperature) of 250°C and a rotation number of 100 rpm for 5 minutes, and then the samples were recovered. The samples were subjected to compression molding into a plate shape (50 mm in length×50 in widthx2 mm in thickness) using a hot compression molding machine, and slowly cooled to provide resin composites. Table 1 shows the raw material composition and evaluation results of each of the resin composites.

<Comparative Example A4>

100 g of the inorganic particles F1 were loaded into an autoclave with a stirrer, and heated to 200°C while being fluidized by stirring. Next, the inside of the autoclave was purged with nitrogen gas, and then 1.4 g of the surface treatment agent S11 were sprayed. The mixture was stirred for 1 hour, and then the inside of the system was purged with nitrogen to provide surface-treated inorganic particles.

Subsequently, a resin composite was obtained by the same method as that of Comparative Example A1 except that: the thermoplastic resin P2 was used in place of the thermoplastic resin P1; the blending amount of the inorganic particles was set to a condition shown in Table 2; and the test temperature (melting temperature) was set to 370°C. Table 2 shows the raw material composition and evaluation results of the resin composite.

<Comparative Examples A5 to 8>

Surface-treated inorganic particles to be used for the production of resin composites were produced in the same manner as in Comparable Example A1 except that the conditions were changed as shown in Table 2. Subsequently, the resin composites were obtained by the same method as that of Example A7. Table 2 shows the raw material composition and evaluation results of each of the resin composites.

<Comparative Example A9>

Surface-treated inorganic particles to be used for the production of a resin composite were produced in the same manner as in Comparable Example A1 except that the conditions were changed as shown in Table 2.

Next, 100 parts by mass of (A) the thermoplastic resin P3, and 80 parts by mass of (B) the surface-treated inorganic particles were weighed, and loaded into a kneader LABO PLASTOMILL (manufactured by Toyo Seiki Seisaku-sho, Ltd.). Kneading was performed at a test temperature (melting temperature) of 190°C and a rotation number of 100 rpm for 5 minutes, and then the sample was recovered. The sample was subjected to compression molding into a plate shape (50 mm in length×50 in width×2 mm in thickness) using a hot compression molding machine, and slowly cooled to provide a resin composite. Table 2 shows the raw material composition and evaluation results of the resin composite.

<Comparative Example A10>

A resin composite was obtained by the same method as that of Example A10 except that the blending amount of the inorganic particles was set to a condition shown in Table 2. Table 2 shows the raw material composition and evaluation results of the resin composite.

<Comparative Example A11>

The production of a resin composite was attempted by the same method as that of Example A10 except that the blending amount of the inorganic particles was set to a condition shown in Table 2. However, the blending amount of the inorganic particles was so large that kneading was not able to be performed and the resin composite was not able to be obtained. Table 2 shows the raw material composition and evaluation results of the resin composite.

[Table 1]

| | (A) Thermoplastic resin | (B) Surface-treated inorganic particles | | | Blending amount of inorganic particles (part(s) by mass) | Average embedding rate [%] | Bending strength [MPa] | Modulus of elasticity [GPa] | Vickers hardness [Hv] |
| | | Inorganic particles | Surface treatment agent | Surface treatment agent amount [part(s) by mass] | | | | | |
|---|---|---|---|---|---|---|---|---|---|
| Comparable Example A1 | P1 | F1 | S5 | 2.4 | 80 | 58% | 125.3 | - | 25.3 |
| Comparable Example A2 | P1 | F1 | S7 | 2.2 | 80 | 58% | 122.7 | - | 23.6 |
| Comparable Example A3 | P1 | F1 | S8 | 2.3 | 80 | 60% | 125.1 | - | 25.3 |
| Comparable Example A4 | P1 | F1 | S10 | 3.0 | 80 | 56% | 119.8 | - | 17.8 |
| Comparable Example A5 | P1 | F1 | S8 | 2.3 | 40 | 55% | 119.4 | - | - |
| Comparable Example A6 | P1 | F1 | S8 | 2.3 | 150 | 57% | 128.9 | - | - |
| Example A7 | P2 | F1 | S5 | 2.4 | 80 | 73% | 178.4 | - | 39.0 |
| Example A8 | P2 | F1 | S6 | 3.0 | 80 | 75% | 177.9 | - | 37.3 |
| Example A9 | P2 | F1 | S7 | 2.2 | 80 | 69% | 172.4 | - | 37.7 |
| Example A10 | P2 | F1 | S8 | 2.3 | 80 | 71% | 177.7 | - | 40.2 |
| Example A11 | P2 | F1 | S9 | 2.9 | 80 | 65% | 169.8 | - | 35.3 |
| Example A12 | P2 | F1 | S10 | 3.0 | 80 | 62% | 149.3 | - | 36.8 |
| Example A13 | P2 | F2 | S6 | 3.0 | 80 | 67% | 170.8 | - | - |
| Example A14 | P2 | F3 | S6 | 3.0 | 80 | 65% | 174.3 | - | - |
| Example A15 | P2 | F4 | S6 | 3.0 | 80 | 64% | 160.1 | - | - |
| Example A16 | P2 | F2 | S8 | 2.3 | 80 | 65% | 173.6 | - | - |
| Example A17 | P2 | F3 | S8 | 2.3 | 80 | 64% | 173.9 | - | - |

| | (A) Thermoplastic resin | (B) Surface-treated inorganic particles | | | Blending amount of inorganic particles (part(s) by mass) | Average embedding rate [%] | Bending strength | Modulus of elasticity | Vickers hardness |
|---|---|---|---|---|---|---|---|---|---|
| | | Inorganic particles | Surface treatment agent | Surface treatment agent amount [part(s) by mass] | | | [MPa] | [GPa] | [Hv] |
| Example A18 | P2 | F4 | S8 | 2.3 | 80 | 61% | 155.3 | - | - |
| Example A19 | P2 | F1 | S8 | 2.3 | 40 | 65% | 175.8 | - | - |
| Example A20 | P2 | F1 | S8 | 2.3 | 60 | 65% | 173.9 | - | - |
| Example A21 | P2 | F1 | S8 | 2.3 | 150 | 66% | 177.8 | - | - |
| Example A22 | P2 | F7 | S7 | 0.5 | 40 | 56% | 144.2 | 5.6 | 28.9 |
| Example A23 | P2 | F7 | S7 | 0.5 | 60 | 60% | 153.7 | 7.2 | 31.6 |
| Example A24 | P2 | F7 | S7 | 0.5 | 100 | 65% | 176.2 | 11.1 | 41.6 |
| Example A25 | P2 | F7 | S7 | 0.5 | 200 | 67% | 167.8 | 12.7 | 46.1 |
| Example A26 | P4 | F7 | S7 | 0.5 | 60 | 68% | 180.3 | 6.5 | 32.9 |
| Example A27 | P4 | F7 | S7 | 0.5 | 100 | 72% | 192.4 | 11.0 | 36.7 |
| Example A28 | P4 | F6 | S7 | 1.0 | 200 | 73% | 192.5 | 11.5 | 50.3 |
| Example A29 | P2 | F6 | S7 | 1.0 | 150 | 66% | 182.9 | 8.3 | 36.4 |
| Example A30 | P2 | F6 | S7 | 1.0 | 200 | 67% | 185.0 | 10.4 | 48.6 |
| Example A31 | P2 | F6 | S7 | 1.0 | 300 | 68% | 189.3 | 13.5 | 55.0 |
| Example A32 | P5 | F6 | S7 | 1.0 | 380 | 63% | 168.1 | 16.9 | 65.3 |

[Table 2]

| | (A) Thermoplastic resin | (B) Surface-treated inorganic particles | | | Blending amount of inorganic particles (part(s) by mass) | Average embedding rate [%] | Bending strength [MPa] | Modulus of elasticity [GPa] | Vickers hardness [Hv] |
|---|---|---|---|---|---|---|---|---|---|
| | | Inorganic particles | Surface treatment agent | Surface treatment agent amount [part(s) by mass] | | | | | |
| Comparative Example A1 | P1 | F1 | S5 | 2.4 | 30 | - | 102.4 | - | 13.2 |
| Comparative Example A2 | P1 | F1 | S12 | 3.4 | 80 | 43% | 72.3 | - | 13.8 |
| Comparative Example A3 | P1 | F1 | S14 | 1.3 | 80 | 45% | 76.1 | - | 14.1 |
| Comparative Example A4 | P2 | F1 | S11 | 1.4 | 80 | 46% | 98.5 | - | 28.2 |
| Comparative Example A5 | P2 | F1 | S12 | 3.4 | 80 | 50% | 87.5 | - | 29.5 |
| Comparative Example A6 | P2 | F1 | S13 | 1.8 | 80 | 51% | 137.3 | - | 31.9 |
| Comparative Example A7 | P2 | F1 | S14 | 1.3 | 80 | 52% | 123.0 | - | 30.8 |
| Comparative Example A8 | P2 | F1 | S15 | 2.4 | 80 | 51% | 124.9 | - | 30.9 |
| Comparative Example A9 | P3 | F5 | S14 | 2.2 | 80 | - | 51.0 | - | - |
| Comparative Example A10 | P2 | F1 | S8 | 2.3 | 30 | - | - | - | 25.8 |
| Comparative Example A11 | P2 | F1 | S8 | 2.3 | 450 | The amount of the inorganic particles was so large that kneading was not able to be performed | | | |

Next, thermoplastic resins used in Examples B1 to B36 and Comparative Examples B1 to B10 and their abbreviations, inorganic particles used therein and their abbreviations, surface treatment agents used therein and their abbreviations, and sample production methods are shown. It should be noted that evaluation methods are the same as the evaluation methods used in Examples A1 to A32 and Comparative Examples A1 to A11.

(Thermoplastic resin)

P1: polybutylene terephthalate (DURANEX 2000, Polyplastics Co., Ltd., melting point: 228°C, melting viscosity at 250°C: 150 Pa·s)

P6: polyamide (Novamid 1010C2, Mitsubishi Engineering-Plastics Corporation, melting point: 215°C, melting viscosity at 240°C: 150 Pa·s)

P2: polyetheretherketone resin (PEEK 1000G, Daicel-Evonik Ltd., melting point: 334°C, melting viscosity at 370°C: 200 Pa·s)

P4: polyetheretherketone resin (PEEK 381G, Victrex, melting point: 334°C, melting viscosity at 370°C: 400 Pa·s)

P5: polyetheretherketone resin (PEEK 90G, Victrex, melting point: 334°C, melting viscosity at 370°C: 100 Pa·s)

(Inorganic particles)

F1: average particle diameter: 0.40 micron, spherical sol-gel silica (Tokuyama Corporation), specific surface area: 7.0 $m^2$/g

F2: average particle diameter: 2.5 microns, crushed silica-zirconia (Tokuyama Corporation), specific surface area: 2.9 $m^2$/g

F3: average particle diameter: 2.8 microns, spherical silica (Tokuyama Corporation), specific surface area: 12 $m^2$/g

F4: average particle diameter: 0.15 micron, spherical silica-zirconia (Tokuyama Corporation), specific surface area: 22 $m^2$/g

F6: average particle diameter: 8.6 microns, zirconia (DAIICHI KIGENSO KAGAKU KOGYO CO., LTD.), aggregate having a primary particle diameter of 1.0 micron, specific surface area: 3.3 $m^2$/g

F7: average particle diameter: 4.0 microns, silica (Tokuyama Corporation), specific surface area: 1.3 $m^2$/g

(Surface treatment agent)

S1: benzoylpropyltrimethoxysilane (BPTMS)

S2: phenoxypropyltrichlorosilane (PPTCS)

S3: 6-phenylhexyldimethylchlorosilane (PHMCS)

S4: 2-hydroxy-4-(3-triethoxysilylpropoxy)diphenyl ketone (HDPKPTES)

S5: 3-styrylpropyltrimethoxysilane (SPTMS)

S6: 3-(N-styrylmethyl-2-aminoethylamino)-propyltrimethoxysilane (SMAPTMS)

S11: hexamethyldisilazane (HMDS)

S12: octadecyltrimethoxysilane (ODTMS)

S13: phenyltrimethoxysilane (PTMS)

S14: vinyltrimethoxysilane (VTMS)

S15: 1-phenyl-1-trichlorosilylbutane (PTCSB)

< Comparable Example B1>

A slurry was obtained by weighing and mixing 100 g of the inorganic particles F1 and 200 ml of toluene and then dispersing the mixture to a primary particle level with a homogenizer. The slurry was loaded into a three-necked flask having set thereon a reflux condenser tube, 2.5 g of the surface treatment agent S1 were added thereto, and the mixture was heated to reflux for 2 hours while being stirred. The solid content was separated with a centrifugal separator, washed twice with toluene, and then dried in a vacuum dryer at 90°C for 10 hours. Thus, surface-treated inorganic particles were obtained.

Next, 100 parts by mass of (A) the thermoplastic resin P1, and 80 parts by mass of (B) the surface-treated inorganic particles were weighed, and loaded into a kneader LABO PLASTOMILL (manufactured by Toyo Seiki Seisaku-sho, Ltd.). Kneading was performed at a test temperature (melting temperature) of 250°C and a rotation number of 100 rpm for 5 minutes, and then the sample was recovered. The sample was subjected to compression molding into a plate shape (50 mm in length×50 in width×2 mm in thickness) using a hot compression molding machine, and slowly cooled to provide a resin composite. Table 3 shows the raw material composition and evaluation results of the resin composite.

< Comparable Example B2 to Example B5>

Surface-treated inorganic particles to be used for the production of resin composites were produced in the same manner as in Comparable Example B1 except that the conditions were changed as shown in Table 3. Subsequently, the resin composites were obtained by the same method as that of Comparable Example B1 except that the blending amount of the inorganic particles was set to a condition shown in Table 3. Table 3 shows the raw material composition and evaluation results of each of the resin composites.

< Comparable Examples B6 to 10>

Surface-treated inorganic particles to be used for the production of resin composites were produced in the same manner as in Comparable Example B1 except that the conditions were changed as shown in Table 3. Subsequently, the resin composites were obtained by the same method as that of Comparable Example A1 except that: the thermoplastic resin P6 was used in place of the thermoplastic resin P1; the blending amount of the inorganic particles was set to a condition shown in Table 3; and the test temperature (melting temperature) was set to 240°C. Table 3 shows the raw material composition and evaluation results of each of the resin composites.

<Examples B11 to 25>

Surface-treated inorganic particles to be used for the production of resin composites were produced in the same manner as in Example Comparable B1 except that the conditions were changed as shown in Table 3. Subsequently, the resin composites were obtained by the same method as that of Comparable Example B1 except that: the thermoplastic resin P2 was used in place of the thermoplastic resin P1; the blending amount of the inorganic particles was set to a condition shown in Table 3; and the test temperature (melting temperature) was set to 370°C. Table 3 shows the raw material composition and evaluation results of each of the resin composites.

<Examples B26>

Surface-treated inorganic particles to be used for the production of a resin composite were produced in the same manner as in Comparable Example B1 except that the conditions were changed as shown in Table 3.

Next, 100 parts by mass of (A) the thermoplastic resin P2, and 150 parts by mass of (B) the surface-treated inorganic particles were weighed, and loaded into a small twin-screw extrusion molding machine (manufactured by Parker Corporation, Inc.). Kneading was performed under the conditions of a test temperature (melting temperature) of 370°C and a rotation number of 500 rpm, and then the sample was recovered. The sample was subjected to compression molding into a plate shape (50 mm in length×50 in width×2 mm in thickness) using a hot compression molding machine, and slowly cooled to provide a resin composite. Table 3 shows the raw material composition and evaluation results of the resin composite.

<Examples B27 to 36>

Surface-treated inorganic particles to be used for the production of resin composites were produced in the same manner as in Comparable Example B1 except that the conditions were changed as shown in Table 3. Subsequently, the resin composites were obtained by the same method as that of Comparable Example B1 except that: the thermoplastic resin used and the blending amount of the inorganic particles were set to conditions shown in Table 3; and the test temperature (melting temperature) was set to 370°C. Table 3 shows the raw material composition and evaluation results of each of the resin composites.

<Comparative Example B1>

Surface-treated inorganic particles to be used for the production of a resin composite were produced in the same manner as in Comparable Example B1 except that the conditions were changed as shown in Table 4.

Next, 100 parts by mass of (A) the thermoplastic resin P1, and 80 parts by mass of (B) the surface-treated inorganic particles were weighed, and loaded into a kneader LABO PLASTOMILL (manufactured by Toyo Seiki Seisaku-sho, Ltd.). Kneading was performed at a test temperature (melting temperature) of 250°C and a rotation number of 100 rpm for 5 minutes, and then the sample was recovered. The sample was subjected to compression molding into a plate shape (50 mm in length×50 in widthx2 mm in thickness) using a hot compression molding machine, and slowly cooled to provide a resin composite. Table 4 shows the raw material composition and evaluation results of the resin composite.

<Comparative Example B2>

Surface-treated inorganic particles to be used for the production of a resin composite were produced in the same manner as in Comparative Example B1 except that the conditions were changed as shown in Table 4. Subsequently, the resin composite was obtained by the same method as that of Comparative Example B1 except that the blending amount of the inorganic particles was set to a condition shown in Table 4. Table 4 shows the raw material composition and evaluation results of the resin composite.

<Comparative Example B3>

Surface-treated inorganic particles to be used for the production of a resin composite were produced in the same manner as in Comparative Example B1 except that the conditions were changed as shown in Table 4. Subsequently, the resin composite was obtained by the same method as that of Comparative Example B1 except that: the thermoplastic resin P6 was used in place of the thermoplastic resin P1; the blending amount of the inorganic particles was set to a condition shown in Table 3; and the test temperature (melting temperature) was set to 240°C. Table 4 shows the raw material composition and evaluation results of the resin composite.

<Comparative Example B4>

100 g of the inorganic particles F1 were loaded into an autoclave with a stirrer, and heated to 200°C while being fluidized by stirring. Next, the inside of the autoclave was purged with nitrogen gas, and then 1.4 g of the surface treatment agent S11 were sprayed. The mixture was stirred for 1 hour, and then the inside of the system was purged with nitrogen to provide surface-treated inorganic particles.

Subsequently, the resin composites were obtained by the same method as that of Comparative Example B1 except that: the thermoplastic resin P2 was used in place of the thermoplastic resin P1; the blending amount of the inorganic particles was set to a condition shown in Table 3; and the test temperature (melting temperature) was set to 360°C. Table 4 shows the raw material composition and evaluation results of the resin composite.

<Comparative Examples B5 to 8>

Surface-treated inorganic particles to be used for the production of resin composites were produced in the same manner as in Comparative Example B1 except that the conditions were changed as shown in Table 4. Subsequently, the resin composites were obtained by the same method as that of Comparative Example B1 except that: the thermoplastic resin P2 was used in place of the thermoplastic resin P1; the blending amount of the inorganic particles was set to a condition shown in Table 4; and the test temperature (melting temperature) was set to 360°C. Table 4 shows the raw material composition and evaluation results of each of the resin composites.

<Comparative Example B9>

A resin composite was obtained by the same method as that of Example B11 except that the blending amount of the inorganic particles was set to a condition shown in Table 4. Table 4 shows the raw material composition and evaluation results of the resin composite.

<Comparative Example B10>

The production of a resin composite was attempted by the same method as that of Example B11 except that the blending amount of the inorganic particles was set to a condition shown in Table 4. However, the blending amount of the inorganic particles was so large that kneading was not able to be performed and the resin composite was not able to be obtained. Table 4 shows the raw material composition and evaluation results of the resin composite.

[Table 3]

| 5 | (A) Thermoplastic resin | (B) Surface-treated inorganic particles | | | Blending amount of inorganic particles (part(s) by mass) | Average embedding rate [%] | Bending strength | Modulus of elasticity | Vickers hardness |
|---|---|---|---|---|---|---|---|---|---|
| | | Inorganic particles | Surface treatment agent | Surface treatment agent amount [part(s) by mass] | | | [MPa] | [GPa] | [Hv] |
| Comparable Example B1 | P1 | F1 | S1 | 2.5 | 80 | 59% | 121.8 | - | 21.7 |
| Comparable Example B2 | P1 | F1 | S3 | 2.3 | 80 | 65% | 127.3 | - | 25.6 |
| Comparable Example B3 | P1 | F1 | S5 | 2.4 | 80 | 66% | 129.1 | - | 23.9 |
| Comparable Example B4 | P1 | F1 | S1 | 2.5 | 40 | 60% | 120.5 | - | - |
| Comparable Example B5 | P1 | F1 | S1 | 2.5 | 150 | 60% | 133.2 | - | - |
| Comparable Example B6 | P6 | F1 | S1 | 2.5 | 80 | 57% | 131.5 | - | 19.3 |
| Comparable Example B7 | P6 | F1 | S3 | 2.3 | 80 | 59% | 132.4 | - | 20.6 |
| Comparable Example B8 | P6 | F1 | S5 | 2.4 | 80 | 58% | 132.1 | - | 20.3 |
| Comparable Example B9 | P6 | F1 | S1 | 2.5 | 40 | 57% | 136.2 | - | - |
| Comparable Example B10 | P6 | F1 | S1 | 2.5 | 150 | 58% | 130.9 | - | - |
| Example B11 | P2 | F1 | S1 | 2.5 | 80 | 68% | 175.2 | - | 36.8 |
| Example B12 | P2 | F1 | S2 | 2.4 | 80 | 67% | 176.4 | - | 37.1 |
| Example B13 | P2 | F1 | S3 | 2.3 | 80 | 72% | 179.4 | - | 38.8 |
| Example B14 | P2 | F1 | S4 | 3.7 | 80 | 66% | 168.2 | - | 36.5 |
| Example B15 | P2 | F1 | S5 | 2.4 | 80 | 73% | 178.4 | - | 39.0 |

(continued)

| 5 | (A) Thermoplastic resin | (B) Surface-treated inorganic particles | | | Blending amount of inorganic particles (part(s) by mass) | Average embedding rate [%] | Bending strength [MPa] | Modulus of elasticity [GPa] | Vickers hardness [Hv] |
|---|---|---|---|---|---|---|---|---|---|
| | | Inorganic particles | Surface treatment agent | Surface treatment agent amount [part(s) by mass] | | | | | |
| Example B16 | P2 | F1 | S6 | 3.0 | 80 | 75% | 177.9 | - | 37.3 |
| Example B17 | P2 | F2 | S3 | 2.3 | 80 | 67% | 175.9 | - | - |
| Example B18 | P2 | F3 | S3 | 2.3 | 80 | 65% | 172.0 | - | - |
| Example B19 | P2 | F4 | S3 | 2.3 | 80 | 62% | 163.3 | - | - |
| Example B20 | P2 | F2 | S6 | 3.0 | 80 | 67% | 170.8 | - | - |
| Example B21 | P2 | F3 | S6 | 3.0 | 80 | 65% | 174.3 | - | - |
| Example B22 | P2 | F4 | S6 | 3.0 | 80 | 64% | 160.1 | - | - |
| Example B23 | P2 | F1 | S1 | 2.5 | 40 | 65% | 170.8 | - | - |
| Example B24 | P2 | F1 | S1 | 2.5 | 60 | 63% | 168.2 | - | - |
| Example B25 | P2 | F1 | S1 | 2.5 | 150 | 64% | 175.3 | - | - |
| Example B26 | P2 | F7 | S1 | 0.5 | 40 | 55% | 142.8 | 5.5 | 27.9 |
| Example B27 | P2 | F7 | S1 | 0.5 | 60 | 57% | 153.6 | 7.2 | 31.3 |
| Example B28 | P2 | F7 | S1 | 0.5 | 100 | 63% | 174.3 | 11.0 | 42.0 |
| Example B29 | P2 | F7 | S1 | 0.5 | 200 | 65% | 167.9 | 12.6 | 47.1 |
| Example B30 | P4 | F7 | S1 | 0.5 | 60 | 71% | 181.3 | 6.6 | 31.9 |
| Example B31 | P4 | F7 | S1 | 0.5 | 100 | 71% | 188.9 | 9.4 | 37.5 |
| Example B32 | P4 | F6 | S1 | 1.2 | 200 | 72% | 189.4 | 11.9 | 49.9 |
| Example B33 | P2 | F6 | S1 | 1.2 | 150 | 70% | 180.7 | 8.3 | 36.7 |
| Example B34 | P2 | F6 | S1 | 1.2 | 200 | 75% | 186.6 | 10.4 | 48.5 |
| Example B35 | P2 | F6 | S1 | 1.2 | 300 | 75% | 188.8 | 13.1 | 54.3 |
| Example B36 | P5 | F6 | S1 | 1.2 | 380 | 63% | 167.7 | 16.9 | 62.7 |

[Table 4]

| | (A) Thermoplastic resin | (B) Surface-treated inorganic particles | | | Blending amount of inorganic particles (part (s) by mass) | Average embedding rate [%] | Bending strength | Modulus of elasticity | Vickers hardness |
|---|---|---|---|---|---|---|---|---|---|
| | | Inorganic particles | Surface treatment agent | Surface treatment agent [part(s) by mass] | | | [MPa] | [GPa] | [Hv] |
| Comparative Example B1 | P1 | F1 | S12 | 3.4 | 80 | 43% | 72.3 | - | 13.8 |
| Comparative Example B2 | P1 | F1 | S13 | 1.8 | 80 | 50% | 101.5 | - | 16.8 |
| Comparative Example B3 | P6 | F1 | S13 | 1.8 | 80 | 47% | 120.2 | - | 18.8 |
| Comparative Example B4 | P2 | F1 | S11 | 1.4 | 80 | 46% | 98.5 | - | 28.2 |
| Comparative Example B5 | P2 | F1 | S12 | 3.4 | 80 | 50% | 87.5 | - | 29.5 |
| Comparative Example B6 | P2 | F1 | S13 | 1.8 | 80 | 51% | 137.3 | - | 31.9 |
| Comparative Example B7 | P2 | F1 | S14 | 1.3 | 80 | 52% | 123.0 | - | 30.8 |
| Comparative Example B8 | P2 | F1 | S15 | 2.4 | 80 | 51% | 124.9 | - | 30.9 |
| Comparative Example B9 | P2 | F1 | S1 | 2.5 | 30 | - | 155.2 | - | 28.2 |
| Comparative Example B10 | P2 | F1 | S1 | 2.5 | 450 | The amount of the inorganic particles was so large that kneading was not able to be performed | | | |

**Claims**

1.  A dental material, comprising a resin composite, which is produced using:

    (A) 100 parts by mass of a thermoplastic resin having a melting point or fluidization temperature of from 300°C to 500°C; and
    (B) 40 parts by mass to 400 parts by mass of inorganic particles surface-treated with a surface treatment agent represented by the following general formula (I),
    the resin composite having an average embedding rate, which is represented by the following equation (1), of 55% or more:

    •General formula (I)         $A\text{-}R^1\text{-}SiR^2mBn$

    in the general formula (I):

    A represents an organic group having a polymerizable carbon-carbon double bond, or an aromatic group;
    $R^1$ represents an organic group having a straight chain moiety constituted of 3 to 12 atoms;
    $R^2$ represents a hydrocarbon group having 1 to 6 carbon atoms;
    B represents an alkoxy group containing a hydrocarbon having 1 to 6 carbon atoms, a halogen group, or an isocyanate group; and
    m and n each represent an integer, a sum of m and n is 3, and m represents an integer of from 0 to 2;

    •Equation (1)      Average embedding rate (%)=100×{EP/(EP+V)}

    in the equation (1):

    EP represents a number {particles/(10 μm×10 μm)} of the inorganic particles per square region measuring 10 μm on each side exposed in a polished surface subjected to platinum vapor deposition treatment in a case where a polished surface formed by subjecting a test piece formed of the resin composite to microtome polishing using a diamond knife is subjected to the platinum vapor deposition treatment and then the polished surface subjected to the platinum vapor deposition treatment is observed with a scanning electron microscope;
    V represents a number {voids/ (10 μm×10 μm)} of voids per square region measuring 10 μm on each side formed through elimination of the inorganic particles in the square region measuring 10 μm on each side where the EP number is counted; and
    the average embedding rate refers to an average value of embedding rates respectively determined at four measurement points.

2.  A dental material according to claim 1, wherein (A) the thermoplastic resin having a melting point or fluidization temperature of from 300°C to 500°C comprises an aromatic polyether ketone resin.

3.  A dental material according to claim 2, wherein the aromatic polyether ketone resin comprises polyetheretherketone.

4.  A dental material according to any one of claims 1 to 3, wherein (B) the surface treatment agent comprises a surface treatment agent represented by the following general formula (II):

    •General formula (II)         $CH_2\text{=}C(R^3)\text{-}COO\text{-}(CH_2)_p\text{-}SiR^2mBn$

    in the general formula (II), $R^3$ represents a hydrogen atom or a methyl group, p represents an integer of from 3 to 12, and $R^2$, B, m, and n have the same meanings as those shown in the general formula (I) .

5.  A dental material according to any one of claims 1 to 4, wherein (B) the inorganic particles surface-treated with the surface treatment agent each have an average particle diameter of 0.05 μm or more and 15 μm or less.

6.  A dental material according to any of claims 1 to 5, wherein the inorganic particles are selected from amorphous silica, borosilicate glass, soda glass, aluminosilicate glass, fluoroaluminosilicate glass, and glass containing a heavy metal (such as barium, strontium); crystallized glass obtained by depositing a crystal in the glass, a glass ceramics such as crystallized glass obtained by depositing a crystal of diopside or leucite; a composite inorganic oxide such as silica-zirconia, silica-titania, or silica-alumina; an oxide obtained by adding an oxide of a Group I metal to the composite oxide; and an inorganic oxide of a metal such as silica, alumina, titania, or zirconia.

7. A method of manufacturing a dental material comprising a resin composite, comprising at least a melt-kneading step of kneading raw materials including:

(A) 100 parts by mass of a thermoplastic resin having a melting point or fluidization temperature of from 300°C to 500°C; and
(B) 40 parts by mass to 400 parts by mass of inorganic particles surface-treated with a surface treatment agent represented by the following general formula (I),
while melting the raw materials by heating in a range of from the melting point or the fluidization temperature or more to 500°C or less:

$$\text{•General formula (I)} \qquad A\text{-}R^1\text{-}SiR^2mBn$$

in the general formula (I):

A represents an organic group having a polymerizable carbon-carbon double bond, or an aromatic group;
$R^1$ represents an organic group having a straight chain moiety constituted of 3 to 12 atoms;
$R^2$ represents a hydrocarbon group having 1 to 6 carbon atoms;
B represents an alkoxy group containing a hydrocarbon having 1 to 6 carbon atoms, a halogen group, or an isocyanate group; and
m and n each represent an integer, a sum of m and n is 3, and m represents an integer of from 0 to 2.

8. A method according to claim 7, wherein (A) the thermoplastic resin having a melting point or fluidization temperature of from 300°C to 500°C comprises an aromatic polyether ketone resin.

9. A method according to claim 8, wherein the aromatic polyether ketone resin comprises polyetheretherketone.

10. A method according to any of claims 7-9, wherein (B) the surface treatment agent comprises a surface treatment agent represented by the following general formula (II):

$$\text{•General formula (II)} \qquad CH_2=C(R^3)\text{-}COO\text{-}(CH_2)_p\text{-}SiR^2mBn$$

in the general formula (II, $R^3$ represents a hydrogen atom or a methyl group, p represents an integer of from 3 to 12, and $R^2$, B, m, and n have the same meanings as those shown in the general formula (I) .

11. A method according to claims 7 or 8, wherein (B) the inorganic particles surface-treated with the surface treatment agent each have an average particle diameter of 0.05 $\mu$m or more and 15 $\mu$m or less.

12. A method according to any of claims 7-11, wherein the inorganic particles are selected from amorphous silica, borosilicate glass, soda glass, aluminosilicate glass, fluoroaluminosilicate glass, and glass containing a heavy metal (such as barium, strontium); crystallized glass obtained by depositing a crystal in the glass, a glass ceramics such as crystallized glass obtained by depositing a crystal of diopside or leucite; a composite inorganic oxide such as silica-zirconia, silica-titania, or silica-alumina; an oxide obtained by adding an oxide of a Group I metal to the composite oxide; and an inorganic oxide of a metal such as silica, alumina, titania, or zirconia.

13. A bone substitute material, comprising a resin composite, which is produced using:

(A) 100 parts by mass of a thermoplastic resin having a melting point or fluidization temperature of from 300°C to 500°C; and
(B) 40 parts by mass to 400 parts by mass of inorganic particles surface-treated with a surface treatment agent represented by the following general formula (I),
the resin composite having an average embedding rate, which is represented by the following equation (1), of 55% or more:

•General formula (I)   $A\text{-}R^1\text{-}SiR^2mBn$

in the general formula (I):

A represents an organic group having a polymerizable carbon-carbon double bond, or an aromatic group;
$R^1$ represents an organic group having a straight chain moiety constituted of 3 to 12 atoms;
$R^2$ represents a hydrocarbon group having 1 to 6 carbon atoms;
B represents an alkoxy group containing a hydrocarbon having 1 to 6 carbon atoms, a halogen group, or an isocyanate group; and
m and n each represent an integer, a sum of m and n is 3, and m represents an integer of from 0 to 2;

•Equation (1)   Average embedding rate $(\%)=100\times\{EP/(EP+V)\}$

in the equation (1):

EP represents a number {particles/(10 $\mu$m$\times$10 $\mu$m)} of the inorganic particles per square region measuring 10 $\mu$m on each side exposed in a polished surface subjected to platinum vapor deposition treatment in a case where a polished surface formed by subjecting a test piece formed of the resin composite to microtome polishing using a diamond knife is subjected to the platinum vapor deposition treatment and then the polished surface subjected to the platinum vapor deposition treatment is observed with a scanning electron microscope;
V represents a number {voids/ (10 $\mu$m$\times$10 $\mu$m)} of voids per square region measuring 10 $\mu$m on each side formed through elimination of the inorganic particles in the square region measuring 10 $\mu$m on each side where the EP number is counted; and
the average embedding rate refers to an average value of embedding rates respectively determined at four measurement points.

14. A method of manufacturing a bone substitute material comprising a resin composite, comprising at least a melt-kneading step of kneading raw materials including:

(A) 100 parts by mass of a thermoplastic resin having a melting point or fluidization temperature of from 300°C to 500°C; and
(B) 40 parts by mass to 400 parts by mass of inorganic particles surface-treated with a surface treatment agent represented by the following general formula (I),
while melting the raw materials by heating in a range of from the melting point or the fluidization temperature or more to 500°C or less:

•General formula (I)   $A\text{-}R^1\text{-}SiR^2mBn$

in the general formula (I):

A represents an organic group having a polymerizable carbon-carbon double bond, or an aromatic group;
$R^1$ represents an organic group having a straight chain moiety constituted of 3 to 12 atoms;
$R^2$ represents a hydrocarbon group having 1 to 6 carbon atoms;
B represents an alkoxy group containing a hydrocarbon having 1 to 6 carbon atoms, a halogen group, or an isocyanate group; and
m and n each represent an integer, a sum of m and n is 3, and m represents an integer of from 0 to 2.

**Patentansprüche**

1. Dentalwerkstoff, umfassend ein Harzkomposit, der hergestellt wird durch Verwendung von:

(A) 100 Masseteilen eines thermoplastischen Harzes mit einem Schmelzpunkt oder einer Fluidisierungstemperatur von 300 °C bis 500 °C; und
(B) 40 Masseteilen bis 400 Masseteilen von mit einem Oberflächenbehandlungsmittel oberflächenbehandelten anorganischen Partikeln, dargestellt durch die folgende allgemeine Formel (I),
wobei das Harzkomposit eine durchschnittliche Einbettungsrate, die durch die folgende Gleichung (1) dargestellt ist, von 55 % oder mehr aufweist:

$$\text{• Allgemeine Formel (I)} \qquad A\text{-}R^1\text{-}SiR^2mBn$$

wobei in der allgemeinen Formel (I):

A eine organische Gruppe mit einer polymerisierbaren Kohlenstoff-Kohlenstoff-Doppelbindung oder eine aromatische Gruppe darstellt;
$R^1$ eine organische Gruppe mit einem aus 3 bis 12 Atomen aufgebauten geradkettigen Teil darstellt;
$R^2$ eine Kohlenwasserstoffgruppe mit 1 bis 6 Kohlenstoffatom(en) darstellt;
B eine Alkoxygruppe, enthaltend einen Kohlenwasserstoff mit 1 bis 6 Kohlenstoffatom(en), eine Halogengruppe oder eine Isocyanatgruppe darstellt; und
m und n jeweils eine ganze Zahl darstellen, eine Summe von m und n 3 ist und m eine ganze Zahl von 0 bis 2 darstellt;

$$\text{• Gleichung (1)} \qquad \text{Durchschnittliche Einbettungsrate (\%)} = 100 \text{ x } \{EP/(EP+V)\}$$

wobei in der Gleichung (1):

EP eine Zahl $\{\text{Partikel}/(10 \, \mu m \text{ x } 10 \, \mu m)\}$ der anorganischen Partikel pro quadratischem Bereich darstellt, messend 10 $\mu$m auf jeder Seite, ausgesetzt in einer polierten Oberfläche, die einer Platinbedampfungsbehandlung in einem Fall unterzogen wird, in dem eine polierte Oberfläche, gebildet durch Unterziehen eines aus dem Harzkomposit gebildeten Teststücks dem Polieren mittels eines Mikrotom-Diamantmessers, der Platinbedampfungsbehandlung unterzogen wird und dann die der Platinbedampfungsbehandlung unterzogene polierte Oberfläche mit einem Rasterelektronenmikroskop beobachtet wird;
V eine Zahl $\{\text{Poren}/(10 \, \mu m \text{ x } 10 \, \mu m)\}$ von Poren pro quadratischem Bereich darstellt, messend 10 $\mu$m auf jeder Seite, gebildet durch Eliminierung der anorganischen Partikel in dem quadratischen Bereich, messend 10 $\mu$m auf jeder Seite, wo die EP-Zahl gezählt wird; und
die durchschnittliche Einbettungsrate sich auf einen durchschnittlichen Wert von Einbettungsraten bezieht bzw. an vier Messpunkten bestimmt wird.

2.  Dentalwerkstoff nach Anspruch 1, wobei (A) das thermoplastische Harz mit einem Schmelzpunkt oder einer Fluidisierungstemperatur von 300 °C bis 500 °C ein aromatisches Polyetherketonharz umfasst.

3.  Dentalwerkstoff nach Anspruch 2, wobei das aromatische Polyetherketonharz Polyetheretherketon umfasst.

4.  Dentalwerkstoff nach einem der Ansprüche 1 bis 3, wobei (B) das Oberflächenbehandlungsmittel ein Oberflächenbehandlungsmittel umfasst, das durch die folgende allgemeine Formel (II) dargestellt ist:

$$\text{• Allgemeine Formel (II)} \qquad CH_2=C(R^3)\text{-}COO\text{-}(CH_2)_p\text{-}SiR^2mBn$$

wobei in der allgemeinen Formel (II) $R^3$ ein Wasserstoffatom oder eine Methylgruppe darstellt, p eine ganze Zahl von 3 bis 12 darstellt und $R^2$, B, m und n die gleichen Bedeutungen wie die in der allgemeinen Formel (I) gezeigten aufweisen.

5.  Dentalwerkstoff nach einem der Ansprüche 1 bis 4, wobei (B) die mit dem Oberflächenbehandlungsmittel oberflächenbehandelten anorganischen Partikel jeweils einen durchschnittlichen Partikeldurchmesser von 0,05 $\mu$m oder mehr und 15 $\mu$m oder weniger aufweisen.

6.  Dentalwerkstoff nach einem der Ansprüche 1 bis 5, wobei die anorganischen Partikel ausgewählt sind aus amorphem Siliziumdioxid, Borsilikatglas, Natronglas, Aluminosilikatglas, Fluoraluminosilikatglas und einem Schwermetall (wie zum Beispiel Barium, Strontium) enthaltenden Glas; kristallisiertem Glas, erhalten durch Ablagerung eines Kristalls in dem Glas, eines glaskeramischen Stoffs, wie zum Beispiel kristallisiertem Glas, erhalten durch Ablagerung eines Diopsid- oder Leucitkristalls; einem anorganischen Kompositoxid, wie zum Beispiel Silika-Zirkonoxid, Silika-Titandioxid oder Silika-Aluminiumoxid; einem Oxid, erhalten durch Zufügen eines Oxids von einem Metall der Gruppe I zum Kompositoxid; und einem anorganischen Oxid von einem Metall, wie zum Beispiel Siliziumdioxid, Aluminiumoxid, Titandioxid oder Zirkonoxid.

7. Verfahren zum Herstellen eines Dentalwerkstoffs, umfassend ein Harzkomposit, umfassend mindestens einen Schmelz-Knet-Schritt zum Kneten von Rohmaterialien, das Folgendes einschließt:

   (A) 100 Masseteile eines thermoplastischen Harzes mit einem Schmelzpunkt oder einer Fluidisierungstemperatur von 300 °C bis 500 °C; und
   (B) 40 Masseteile bis 400 Masseteile von mit einem Oberflächenbehandlungsmittel oberflächenbehandelten anorganischen Partikeln, dargestellt durch die folgende allgemeine Formel (I),
   während des Schmelzens der Rohmaterialien durch Erhitzen in einem Bereich von dem Schmelzpunkt oder der Fluidisierungstemperatur oder mehr bis 500 °C oder weniger:

   $$\text{• Allgemeine Formel (I)} \qquad A\text{-}R^1\text{-}SiR^2mBn$$

   wobei in der allgemeinen Formel (I):

   A eine organische Gruppe mit einer polymerisierbaren Kohlenstoff-Kohlenstoff-Doppelbindung oder eine aromatische Gruppe darstellt;
   $R^1$ eine organische Gruppe mit einem aus 3 bis 12 Atomen aufgebauten geradkettigen Teil darstellt;
   Reine Kohlenwasserstoffgruppe mit 1 bis 6 Kohlenstoffatom(en) darstellt;
   B eine Alkoxygruppe, enthaltend einen Kohlenwasserstoff mit 1 bis 6 Kohlenstoffatom(en), eine Halogengruppe oder eine Isocyanatgruppe darstellt; und
   m und n jeweils eine ganze Zahl darstellen, eine Summe von m und n 3 ist, und m eine ganze Zahl von 0 bis 2 darstellt.

8. Verfahren nach Anspruch 7, wobei (A) das thermoplastische Harz mit einem Schmelzpunkt oder einer Fluidisierungstemperatur von 300 °C bis 500 °C ein aromatisches Polyetherketonharz umfasst.

9. Verfahren nach Anspruch 8, wobei das aromatische Polyetherketonharz Polyetheretherketon umfasst.

10. Verfahren nach einem der Ansprüche 7 bis 9, wobei (B) das Oberflächenbehandlungsmittel ein Oberflächenbehandlungsmittel umfasst, das durch die folgende allgemeine Formel (II) dargestellt ist:

    $$\text{• Allgemeine Formel (II)} \qquad CH_2{=}C(R^3)\text{-}COO\text{-}(CH_2)_p\text{-}SiR^2mBn$$

    wobei in der allgemeinen Formel (II) $R^3$ ein Wasserstoffatom oder eine Methylgruppe darstellt, p eine ganze Zahl von 3 bis 12 darstellt und $R^2$, B, m und n die gleichen Bedeutungen wie die in der allgemeinen Formel (I) gezeigten aufweisen.

11. Verfahren nach den Ansprüchen 7 oder 8, wobei (B) die mit dem Oberflächenbehandlungsmittel oberflächebehandelten anorganischen Partikel jeweils einen durchschnittlichen Partikeldurchmesser von 0,05 $\mu$m oder mehr und 15 $\mu$m oder weniger aufweisen.

12. Verfahren nach einem der Ansprüche 7 bis 11, wobei die anorganischen Partikel ausgewählt sind aus amorphem Siliziumdioxid, Borsilikatglas, Natronglas, Aluminosilikatglas, Fluoraluminosilikatglas und einem Schwermetall (wie zum Beispiel Barium, Strontium) enthaltenden Glas; kristallisiertem Glas, erhalten durch Ablagerung eines Kristalls in dem Glas, eines glaskeramischen Stoffs, wie zum Beispiel kristallisiertem Glas, erhalten durch Ablagerung eines Diopsid- oder Leucitkristalls; einem anorganischen Kompositoxid, wie zum Beispiel Silika-Zirkonoxid, Silika-Titandioxid oder Silika-Aluminiumoxid; einem Oxid, erhalten durch Zufügen eines Oxids von einem Metall der Gruppe I zum Kompositoxid; und einem anorganischen Oxid von einem Metall, wie zum Beispiel Siliziumdioxid, Aluminiumoxid, Titandioxid oder Zirkonoxid.

13. Knochenersatzmaterial, umfassend ein Harzkomposit, das hergestellt wird durch Verwendung von:

    (A) 100 Masseteilen eines thermoplastischen Harzes mit einem Schmelzpunkt oder einer Fluidisierungstemperatur von 300 °C bis 500 °C; und

(B) 40 Masseteilen bis 400 Masseteilen von mit einem Oberflächenbehandlungsmittel oberflächenbehandelten anorganischen Partikeln, dargestellt durch die folgende allgemeine Formel (I),

wobei das Harzkomposit eine durchschnittliche Einbettungsrate, die durch die folgende Gleichung (1) dargestellt ist, von 55 % oder mehr aufweist:

$$\bullet \text{ Allgemeine Formel (I)} \qquad A\text{-}R^1\text{-}SiR^2mBn$$

wobei in der allgemeinen Formel (I):

A eine organische Gruppe mit einer polymerisierbaren Kohlenstoff-Kohlenstoff-Doppelbindung oder eine aromatische Gruppe darstellt;

$R^1$ eine organische Gruppe mit einem aus 3 bis 12 Atomen aufgebauten geradkettigen Teil darstellt;

Reine Kohlenwasserstoffgruppe mit 1 bis 6 Kohlenstoffatom(en) darstellt;

B eine Alkoxygruppe, enthaltend einen Kohlenwasserstoff mit 1 bis 6 Kohlenstoffatom(en), eine Halogengruppe oder eine Isocyanatgruppe darstellt; und

m und n jeweils eine ganze Zahl darstellen, eine Summe von m und n 3 ist und m eine ganze Zahl von 0 bis 2 darstellt;

$$\bullet \text{ Gleichung (1)} \qquad \text{Durschnittliche Einbettungsrate } (\%) = 100 \text{ x } \{EP/(EP+V)\}$$

wobei in der Gleichung (1):

EP eine Zahl {Partikel/(10 $\mu$m x 10 $\mu$m)} der anorganischen Partikel pro quadratischem Bereich darstellt, messend 10 $\mu$m auf jeder Seite, ausgesetzt in einer polierten Oberfläche, die einer Platinbedampfungsbehandlung in einem Fall unterzogen wird, in dem eine polierte Oberfläche, gebildet durch Unterziehen eines aus dem Harzkomposit gebildeten Teststücks dem Polieren mittels eines Mikrotom-Diamantmessers, der Platinbedampfungsbehandlung unterzogen wird und dann die der Platinbedampfungsbehandlung unterzogene polierte Oberfläche mit einem Rasterelektronenmikroskop beobachtet wird;

V eine Zahl {Poren/(10 $\mu$m x 10 $\mu$m)} von Poren pro quadratischem Bereich darstellt, messend 10 $\mu$m auf jeder Seite, gebildet durch Eliminierung der anorganischen Partikel in dem quadratischen Bereich, messend 10 $\mu$m auf jeder Seite, wo die EP-Zahl gezählt wird; und

die durchschnittliche Einbettungsrate sich auf einen durchschnittlichen Wert von Einbettungsraten bezieht bzw. an vier Messpunkten bestimmt wird.

14. Verfahren zum Herstellen eines Knochenersatzmaterials, umfassend ein Harzkomposit, umfassend mindestens einen Schmelz-Knet-Schritt zum Kneten von Rohmaterialien, das Folgendes einschließt:

(A) 100 Masseteile eines thermoplastischen Harzes mit einem Schmelzpunkt oder einer Fluidisierungstemperatur von 300 °C bis 500 °C; und

(B) 40 Masseteile bis 400 Masseteile von mit einem Oberflächenbehandlungsmittel oberflächenbehandelten anorganischen Partikeln, dargestellt durch die folgende allgemeine Formel (I),

während des Schmelzens der Rohmaterialien durch Erhitzen in einem Bereich von dem Schmelzpunkt oder der Fluidisierungstemperatur oder mehr bis 500 °C oder weniger:

$$\bullet \text{ Allgemeine Formel (I)} \qquad A\text{-}R^1\text{-}SiR^2mBn$$

wobei in der allgemeinen Formel (I):

A eine organische Gruppe mit einer polymerisierbaren Kohlenstoff-Kohlenstoff-Doppelbindung oder eine aromatische Gruppe darstellt;

$R^1$ eine organische Gruppe mit einem aus 3 bis 12 Atomen aufgebauten geradkettigen Teil darstellt;

Reine Kohlenwasserstoffgruppe mit 1 bis 6 Kohlenstoffatom(en) darstellt;

B eine Alkoxygruppe, enthaltend einen Kohlenwasserstoff mit 1 bis 6 Kohlenstoffatom(en), eine Halogengruppe oder eine Isocyanatgruppe darstellt; und

m und n jeweils eine ganze Zahl darstellen, eine Summe von m und n 3 ist und m eine ganze Zahl von 0 bis 2 darstellt.

## Revendications

1. Matériau dentaire comprenant un composite de résine, qui est produit en utilisant :

   (A) 100 parties en masse d'une résine thermoplastique ayant un point de fusion ou une température de fluidisation allant de 300 °C à 500 °C ; et
   (B) 40 parties en masse à 400 parties en masse de particules inorganiques traitées en surface avec un agent de traitement de surface représenté par la formule générale (I) suivante,
   le composite de résine ayant un taux d'inclusion moyen, qui est représenté par l'équation (I) suivante, de 55 % ou supérieur :

   $$\text{*Formule générale (I)} \qquad A\text{-}R^1\text{-}SiR^2mBn$$

   dans la formule générale (I) :

   A représente un groupe organique ayant une double liaison carbone-carbone polymérisable ou un groupe aromatique ;
   $R^1$ représente un groupe organique ayant une partie de chaîne linéaire constituée de 3 à 12 atomes ;
   $R^2$ représente un groupe hydrocarbure portant 1 à 6 atomes de carbone ;
   B représente un groupe alcoxy contenant un hydrocarbure portant 1 à 6 atomes de carbone, un groupe halogène ou un groupe isocyanate ; et
   m et n représentent chacun un nombre entier, une somme de m et de n vaut 3 et m représente un nombre entier valant de 0 à 2 ;

   $$\text{*Équation (1) Taux d'inclusion moyen (\%)} = 100 \times \{ EP / (EP + V) \}$$

   dans l'équation (1) :

   EP représente un nombre { particules/ (10 $\mu$m x 10 $\mu$m) } des particules inorganiques par zone carrée mesurant 10 $\mu$m sur chaque côté exposé dans une surface polie soumise à un traitement par dépôt de vapeur de platine dans un cas où une surface polie formée par la soumission d'une pièce d'essai formée du composite de résine à un polissage microtome en utilisant un couteau diamant est soumise au traitement par dépôt de vapeur de platine et la surface polie soumise au traitement par dépôt de vapeur est ensuite observée avec un microscope électronique à balayage ;
   V représente un nombre de vides par zone carrée (vides / (10 $\mu$m x 10 $\mu$m) mesurant 10 $\mu$m sur chaque côté formé par l'élimination des particules inorganiques dans la zone carrée mesurant 10 $\mu$m sur chaque côté où le nombre EP est compté ; et
   le taux d'inclusion moyen se réfère à une valeur moyenne de taux d'inclusion déterminée respectivement à quatre points de mesure.

2. Matériau dentaire selon la revendication 1, dans lequel (A) la résine thermoplastique ayant un point de fusion ou une température de fluidisation allant de 300 °C à 500 °C comprend une résine de polyéther cétone aromatique.

3. Matériau dentaire selon la revendication 2, dans lequel la résine de polyéther cétone aromatique comprend du polyétheréthercétone.

4. Matériau dentaire selon l'une quelconque des revendications 1 à 3, dans lequel (B) l'agent de traitement de surface comprend un agent de traitement de surface représenté par la formule générale (II) suivante :

   $$\text{*Formule générale (II)} \qquad CH_2 = C(R^3)\text{-}COO\text{-}(CH_2)_p\text{-}SiR^2mBn$$

   dans la formule générale (II), $R^3$ représente un atome d'hydrogène ou un groupe méthyle, p représente un nombre entier valant de 3 à 12, et $R^2$, B, m et n ont les mêmes significations que celles illustrées dans la formule générale (I).

**5.** Matériau dentaire selon l'une quelconque des revendications 1 à 4, dans lequel (B) les particules inorganiques traitées en surface avec l'agent de traitement en surface ont chacune un diamètre de particule moyen de 0,05 $\mu$m ou supérieur et de 15 $\mu$m ou inférieur.

**6.** Matériau dentaire selon l'une quelconque des revendications 1 à 5, dans lequel les particules inorganiques sont sélectionnées parmi la silice amorphe, le verre borosilicaté, le verre de soude, le verre d'aluminosilicate, le verre de fluoroaluminosilicate et le verre contenant un métal lourd (tel que le baryum, le strontium) ; le verre cristallisé obtenu par dépôt d'un cristal dans le verre, une vitrocéramique telle que le verre cristallisé obtenu par dépôt d'un cristal de diopside ou de leucite ; un oxyde inorganique composite tel que le silice-zircone, le silice-titane ou le silice-alumine ; un oxyde obtenu par l'addition d'un oxyde d'un métal du groupe I à l'oxyde composite ; et un oxyde inorganique d'un métal tel que le silice, l'alumine, le titane ou le zircone.

**7.** Procédé de fabrication d'un matériau dentaire comprenant un composite de résine, comprenant au moins une étape de malaxage à l'état fondu consistant à malaxer des matériaux bruts consistant en :

   (A) 100 parties en masse d'une résine thermoplastique ayant un point de fusion ou une température de fluidisation allant de 300 °C à 500 °C ; et
   (B) 40 parties en masse à 400 parties en masse de particules inorganiques traitées en surface avec un agent de traitement de surface représenté par la formule générale (I) suivante,
   tout en faisant fondre les matériaux bruts par chauffage à une température comprise entre le point de fusion ou la température de fluidisation ou plus jusqu'à 500 °C ou moins :

   *Formule générale (I)        $A-R^1-SiR^2mBn$

   dans la formule générale (I) :

   A représente un groupe organique ayant une double liaison carbone-carbone polymérisable ou un groupe aromatique ;
   $R^1$ représente un groupe organique ayant une partie de chaîne linéaire constituée de 3 à 12 atomes ;
   $R^2$ représente un groupe hydrocarbure portant 1 à 6 atomes de carbone ;
   B représente un groupe alcoxy contenant un hydrocarbure portant 1 à 6 atomes de carbone, un groupe halogène ou un groupe isocyanate ; et
   m et n représentent un nombre entier, une somme de m et n vaut 3, et m représente un nombre entier valant de 0 à 2.

**8.** Procédé selon la revendication 7, dans lequel (A) la résine thermoplastique ayant un point de fusion ou une température de fluidisation allant de 300 °C à 500 °C comprend une résine de cétone de polyéther aromatique.

**9.** Procédé selon la revendication 8, dans lequel la résine de cétone de polyéther aromatique comprend du polyétheréthercétone.

**10.** Procédé selon l'une quelconque des revendications 7 à 9, dans lequel (B) l'agent de traitement de surface comprend un agent de traitement de surface représenté par la formule générale (II) suivante :

   *Formule générale (II)        $CH_2 = C(R^3)-COO-(CH_2)_p -SiR^2mBn$

   dans la formule générale (II), $R^2$ représente un atome d'hydrogène ou un groupe méthyle, p représente un nombre entier valant de 3 à 12, et $R^2$, B, m, et n ont les mêmes significations que celle illustrées dans la formule générale (I).

**11.** Procédé selon les revendications 7 ou 8, dans lequel (B) les particules inorganiques traitées en surface par l'agent de traitement de surface ont chacune un diamètre de particule moyen de 0,05 $\mu$m ou supérieur et de 15 $\mu$m ou inférieur.

**12.** Procédé selon l'une quelconque des revendications 7 à 11, dans lequel les particules inorganiques sont sélectionnées parmi la silice amorphe, le verre borosilicaté, le verre de soude, le verre d'aluminosilicate, le verre de fluoroaluminosilicate et le verre contenant un métal lourd (tel que le baryum, le strontium) ; le verre cristallisé obtenu par dépôt d'un cristal dans le verre, une vitrocéramique telle que le verre cristallisé obtenu par dépôt d'un cristal de diopside ou de leucite ; un oxyde inorganique composite tel que le silice-zircone, le silice-titane ou le silice-alumine ; un oxyde obtenu par l'addition d'un oxyde d'un métal du groupe I à l'oxyde composite ; et un oxyde inorganique d'un métal tel que le silice, l'alumine, le titane ou le zircone.

**13.** Substitut osseux comprenant un composite de résine, qui est produit en utilisant :

(A) 100 parties en masse d'une résine thermoplastique ayant un point de fusion ou une température de fluidisation allant de 300 °C à 500 °C ; et
(B) 40 parties en masse à 400 parties en masse de particules inorganiques traitées en surface par un agent de traitement de surface représenté par la formule générale (I) suivante,
le composite de résine ayant un taux d'inclusion moyen, qui est représenté par l'équation (1) suivante, de 55% ou supérieur :

$$\text{*Formule générale (I)} \quad \text{A-R}^1\text{-SiR}^2\text{mBn}$$

dans la formule générale (I) :

A représente un groupe organique ayant une double liaison carbone-carbone polymérisable ou un groupe aromatique ;
$R^1$ représente un groupe organique ayant une partie de chaîne linéaire constituée de 3 à 12 atomes ;
$R^2$ représente un groupe hydrocarbure portant 1 à 6 atomes de carbone ;
B représente un groupe alcoxy contenant un hydrocarbure portant 1 à 6 atomes de carbone, un groupe halogène ou un groupe isocyanate ; et
m et n représentent un nombre entier, une somme de m et n vaut 3, et m représente un nombre entier valant de 0 à 2.

$$\text{*Équation (1)} \quad \text{Taux d'inclusion moyen} \quad (\%) = 100 \times \{ EP / (EP + V) \}$$

dans l'équation (1) :

EP représente un nombre { particules / (10 $\mu$m x 10 $\mu$m) } des particules inorganiques par zone carrée mesurant 10 $\mu$m sur chaque côté exposé dans une surface polie soumise à un traitement par dépôt de vapeur de platine dans un cas où une surface polie formée par la soumission d'une pièce d'essai formée du composite de résine à un polissage microtome en utilisant un couteau diamant est soumise au traitement par dépôt de vapeur et la surface polie soumise au traitement par dépôt de vapeur est ensuite observée avec un microscope électronique à balayage ;
V représente un nombre de vides par zone carrée (vides / (10 $\mu$m x 10 $\mu$m) mesurant 10 $\mu$m sur chaque côté formé par l'élimination des particules inorganiques dans la zone carrée mesurant 10 $\mu$m sur chaque côté où le nombre EP est compté ; et
le taux d'inclusion moyen se réfère à une valeur moyenne des taux d'inclusion déterminée respectivement à quatre points de mesure.

**14.** Procédé de fabrication d'un substitut osseux comprenant un composite de résine, comprenant au moins une étape de malaxage à l'état fondu consistant à malaxer des matériaux bruts contenant :

(A) 100 parties en masse d'une résine thermoplastique ayant un point de fusion ou une température de fluidisation allant de 300 °C à 500 °C ; et
(B) 40 parties en masse à 400 parties en masse de particules inorganiques traitées en surface par un agent de traitement de surface représenté par la formule générale (I) suivante,
tout en faisant fondre les matériaux bruts par chauffage à une température comprise entre le point de fusion ou la température de fluidisation ou plus à 500 °C ou moins :

$$\text{*Formule générale (I)} \quad \text{A-R}^1\text{-SiR}^2\text{mBn}$$

dans la formule générale (I) :

A représente un groupe organique ayant une double liaison carbone-carbone polymérisable ou un groupe aromatique ;

$R^1$ représente un groupe organique ayant une partie de chaîne linéaire constituée de 3 à 12 atomes ;

$R^2$ représente un groupe hydrocarbure portant 1 à 6 atomes de carbone ;

B représente un groupe alcoxy contenant un hydrocarbure portant 1 à 6 atomes de carbone, un groupe halogène ou un groupe isocyanate ; et

m et n représentent chacun un nombre entier, une somme de m et n vaut 3, et m représente un nombre entier valant de 0 à 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8012305 A
- WO 2002005752 A1
- JP 2005170813 A
- JP 10130116 A
- JP 2005330378 A
- JP 2008266577 A
- JP 2008137854 A
- JP 2003171120 A
- JP 2005325296 A
- JP 58110414 A
- JP 58156524 A
- JP 2008007381 A